0317649

INTERNATIONAL FORM — BUDAPEST TREATY ON THE INTERNATIONAL RECOGNITION OF THE DEPOSIT OF MICRO-ORGANISMS FOR THE PURPOSES OF PATENT PROCEDURE

RECEIPT IN THE CASE OF AN ORIGINAL DEPOSIT
issued pursuant to Rule 7.1 by the
INTERNATIONAL DEPOSITARY AUTHORITY
identified at the bottom of this
page.

Depositor Name : TEIJIN LIMITED

Representative: Sashiro Okamoto (and one other)

Address : 11, Minamihonmachi 1-chome, Higashi-ku, Osaka-shi Osaka, Japan

| I. Indication for Microorganism |
| --- |

| (Indication for Identification by Depositor) | (Deposit Number) Bikoken Joki No. 1909 |
| --- | --- |
| Escherichia coli HB101 [pEAP8] | (FERM BP-1909) |

| II. Scientific Properties and Taxonomic Designation |
| --- |

The Microorganism in the above I is accompanied by a document in which the following matter(s) is described:

[ x ] Scientific Properties    [ x ] Taxonomic Designation

| III. Receipt and Deposit |
| --- |

This International Depositary Authority accepts the deposit of the microorganism in the above I which said Authority received on June 10, 1988.

| IV. International Depositary Authority |
| --- |

Name : Fermentation Research Institute
Agency of Industrial Science and Technology
Tomoo Suzuki

DIRECTOR GENERAL

Address: 1-3, Higashi 1 chome Tsukuba-shi Ibaraki-ken 305, Japan

June 10, 1988

Depositor except the aforementioned

    Name:  Rikagaku Kenkyusho
    Representative:  Minoru Oda
    Address:  2-1, Hirosawa, Wako-shi, Saitama, Japan

Europäisches Patentamt

European Patent Office

Office européen des brevets

(11) Publication number: **0 317 649**
**A1**

## EUROPEAN PATENT APPLICATION

published in accordance with Art. 158(3) EPC

(21) Application number: 88905436.7

(22) Date of filing: 17.06.88

Data of the international application taken as a basis:

(86) International application number:
PCT/JP88/00595

(87) International publication number:
WO88/10302 (29.12.88 88/28)

(51) Int. Cl.³: **C 12 N 15/00**
C 12 N 1/20, C 12 P 21/02
C 07 K 13/00
//(C12N1/20, C12R1:19),
(C12P21/02, C12R1:19)

(30) Priority: 17.06.87 JP 152353/87

(43) Date of publication of application:
31.05.89 Bulletin 89/22

(84) Designated Contracting States:
CH DE FR GB LI

(71) Applicant: TEIJIN LIMITED
11 Minamihonmachi 1-chome Higashi-ku
Osaka-shi Osaka 541(JP)

(71) Applicant: RIKAGAKU KENKYUSHO
2-1 Hirosawa
Wako-shi Saitama-ken351-01(JP)

(72) Inventor: NAKAMURA, Satoshi
18-4, Tamadaira 3-chome Hino-shi
Tokyo 191(JP)

(72) Inventor: ICHIKAWA, Yataro
11-7, Kotesashi-cho 2-chome Tokorozawa-shi
Saitama 359(JP)

(72) Inventor: HORIKOSHI, Koki
39-8, Sakuradai 4-chome Nerima-ku
Tokyo 176(JP)

(74) Representative: Votier, Sidney David et al,
CARPMAELS & RANSFORD 43, Bloomsbury Square
London WC1A 2RA(GB)

(54) NOVEL PLASMID, MICROBIAL CELLS, PROCESS FOR PREPARING POLYPEPTIDE WITH ONCOSTATIC ACTIVITY, AND SECRETED POLYPEPTIDE WITH ONCOSTATIC ACTIVITY.

(57) A plasmid containing: (1) a first DNA region having a DNA sequence coding a polypeptide with an oncostatic activity, a DNA sequence having the promoter function of controlling expression of the polypeptide, and a DNA sequence coding a signal peptide, and (2) a second DNA region having a DNA sequence giving host cells the action of accelerating extracellular secretion and a DNA sequence having the promoter function of controlling expression of the DNA sequence; microbial cells transformed by said plasmid; a process for producing a polypeptide with an oncostatic activity through extracellular secretion using said cells; and a polypeptide with an oncostatic activity secreted from said cells are disclosed.

Fig. 20

– 1 –

## DESCRIPTION

TITLE OF THE INVENTION

Novel Plasmid, Microbial Cell, Process for
Production of Polypeptide Having Antitumor
Activity, and Secretory Polypeptide Having
Antitumor Activity

TECHNICAL FIELD

The present invention relates to novel recombinant plasmid containing a DNA region coding for a polypeptide having antitumor activity, a novel recombinant microbial cell transformed with the plasmid, a process for production of the polypeptide having antitumor activity by using the microbial cell to secrete the polypeptide outside the cell, and the secretory polypeptide having an antitumor activity.

In the specification, amino acids and peptides are expressed by the abbreviation adopted by the IUPAC-IUB Committee on Biochemical Nomenclature (CBN). For example, the following abbreviations are used.

Ala L-alanine
Arg L-arginine
Asn L-asparagine
Asp L-aspartic acid
Cys L-cysteine
Gln L-glutamine
Glu L-glutamic acid
Gly glycine
His L-histidine
Ile L-isoleucine
Leu L-leucine
Lys L-lysine
Met L-methionine
Phe L-phenylalanine
Pro L-proline
Ser L-serine
Thr L-threonine

Trp   L-tryptophan

Tyr   L-tyrosine

Val   L-valine

Further, DNA sequences are expressed by an abbreviation of the base contained in each deoxyribonucleotide composing the DNA. For example, the following abbreviations are used.

A      adenine (denotes deoxyadenylic acid)

C      cytosine (denotes deoxycytidylic acid)

G      granine (denotes deoxyguanylic acid)

T      thymine (denotes deoxythymidylic acid)

Furthermore, $(H_2N)$- and -(COOH) represent an amino terminus and a carboxy terminus of an amino acid sequence, respectively, and (5')- and -(3') represent 5'-terminus and 3-'terminus of a DNA sequence, respectively.

BACKGROUND ART

Carswell et al. found that a serum taken from a mouse, after an administration of endotoxin to the mouse which had been previously stimulated with Bacillus Calmette-Guerin (BCG) or the like, contains a substance which can result in hemorrhagic necrosis of a tumor caused from transplanted Meth A sarcoma, and designated the substance as Tumor Necrosis Factor (hereinafter referred to as TNF) [E.A. Carswell, et al., Proc. Natl. Acad. Sci., USA, 72, 3666 (1975)]. The TNF exists in many animals such as mice, rabbits, and humans, exhibits the activity thereof specifically to tumor cells regardless of species, and is thought to be useful as an antitumor agent.

Recently, Pennica et al. cloned the cDNA of a human TNF, determined the primary structure of a human-TNF protein, and reported an expression of the human-TNF gene in Escherichia coli [D. Pennica, et al., Nature, 312, 724 (1984)]. Thereafter, Shirai et al., [T. Shirai, et al., Nature, 313, 803 (1985)], Somura et al., [Somura, et al., Gan To Kagaku Ryoho (Cancer and Chemotherapy), 12, 160, (1985)], Wang et al. [A.M. Wang, et al., Science, 228, 149 (1985)], and Marmenout, et al., [A. Marmenout, et al., Eur J. Biochem., 152, 515 (1985)], all reported the expression of an intact human TNF gene or the human TNF gene without two amino acids at the amino terminus thereof, in Escherichia coli.

By using the above recombinant DNA technique, it became possible to obtain an intact pure human-TNF protein and TNF protein without two amino acids at the amino terminus, in quantity, and these were subjected to clinical tests. However, the estimated effects for those of the native TNF were not observed [Haranaka, BIO medica, 3, 343 (1988)].

Recently, the development of recombinant DNA techniques has made it possible to produce various kinds of useful proteins in quantity, by using microorganisms or the like, and a production by using Escherichia coli is considered most convenient. In this case, however, useful proteins are usually accumulated within cells, and considerable defects occur in the properties of some of these proteins.

Proteins hitherto attempted to be accumulated in cells include interferon-γ (INF-γ), interleukin 2 (IL2), human growth hormone, or the like. When INF-γ is accumulated in cells, an inclusion body is formed and the activity thereof sometimes is not exhibited. In the case of IL2, for example, S-S bonds are formed out of position, and thus the activity thereof sometimes is not exhibited. Further, in the case of the human growth hormone, sometimes Met remains at the amino terminus.

On the other hand, when TNF is accumulated in cells, the formation of an inclusion body is not observed, the formation of S-S bonds out of position does not occur, and particularly, the processing of Met is correct, for example, in the polypeptide wherein two amino acids are deleted at the amino terminus thereof [M. Yamada, et al., J. Biotechnol, 3, 141, (1985)].

However, as explained above [Haranaka, BIO medica, 3, 343 (1988)], the effect thereof in vivo is lower than that of the native TNF, and the behavior thereof is considerably different from those of other proteins.

After conducting research projects on the production in cells and extracellular secretion of the polypeptide having antitumor activity, the inventors of the present invention surprisingly found that, by secreting the polypeptide having an antitumor activity out of cells, the activity thereof can be greatly improved, although the reasons therefor are not clear.

The object of the present invention is to produce the polypeptide having an antitumor activity by Escherichia coli or the like wherein the polypeptide is produced in the form of one having an enhanced activity by extracellular secretion, and thus to provide a DNA region which contains a DNA sequence coding for the polypeptide having an antitumor activity and can cause extracellular secretion, and to provide a novel recombinant plasmid to which the region is incorporated.

Another object of the present invention is to provide a novel recombinant microbial cell which is transformed with the novel recombinant plasmid and is capable of producing the desired polypeptide having an antitumor activity, out of the cell.

Still another object of the present invention is to provide a process for production of the polypeptide having antitumor activity by extracellular secretion, using the microbial cell. Still another object of the present invention is to provide the polypeptide having an antitumor activity, which is secreted from the microbial cell.

Other objects of the present invention will be apparent from the following description.

DISCLOSURE OF INVENTION

The present invention relates to a plasmid comprising

(1)  a first DNA region comprising a DNA sequence coding for a polypeptide having an antitumor activity, a DNA sequence having a promoter function for controlling an expression of said polypeptide, and a DNA sequence coding for a signal peptide, and

(2)  a second DNA region comprising a DNA sequence causing host cells to facilitate extracellular secretion, and a DNA sequence having a promoter function for controlling an expression of said DNA sequence, a microbial cell transformed with the plasmid, a process for production of the polypeptide having an antitumor activity by secreting the same outside the cell using the microbial cell, and the polypeptide having an antitumor activity which is secreted from the microbial cell.

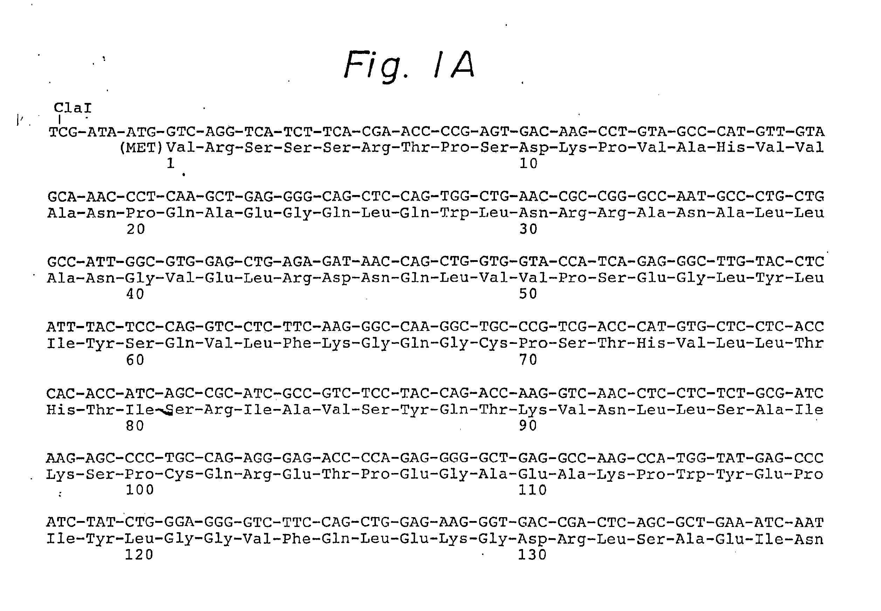

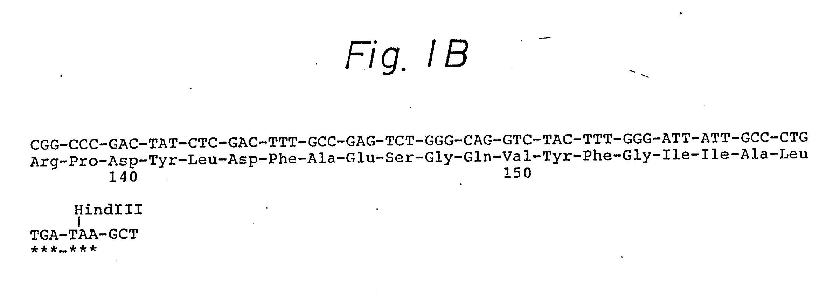

In the present invention, the antitumor-active polypeptide includes the polypeptide containing the amino acid sequence shown in Fig. 1, and polypeptide containing an amino acid sequence wherein one or more amino acid groups are replaced in, deleted from or inserted to the amino acid sequence shown in Fig. 1. Therefore, the polypeptide according to the present invention includes human TNF, mouse TNF, bovine TNF, rabbit TNF, and modified products thereof.

Further, the present polypeptide having an antitumor activity also includes the modified human TNF already reported, wherein the human TNF protein shown in Fig. 1 is modified to the extent that the antitumor activity is not lost or enhanced; for example, modified polypeptide wherein 1, 2, 3, 4, 5, 6, 7, 8, 9, 10 or 11 amino acids at the amino terminus are deleted; modified polypeptides wherein Glu is substituted for $Val^1$; Glu is substituted for $Arg^2$, Asn or Cys is substituted for $Ser^4$, Asp is substituted for $Ser^5$, Lys is substituted for $Arg^6$, His or Ile is substituted for $Thr^7$, Ser or Arg is substituted for $Pro^8$, Thr or Lys is substituted for $Ser^9$, Leu or Arg is substituted for $Asp^{13}$, $Lys^{11}$ is

deleted, Ala is substituted for $Val^{15}$, Leu is substituted for $Val^{16}$, Ile is substituted for $Val^{17}$, Gly, Ile, Ser, Thr, Val, Leu or Pro is substituted for $Ala^{18}$, His is substituted for $Pro^{20}$, Val is substituted for $Ala^{22}$, Glu is substituted for $Gly^{24}$, Asn is substituted for $Arg^{31}$, His, Ala, Glu or Thr is substituted for $Arg^{32}$, Asp is substituted for $Ala^{33}$, Arg is substituted for $Asn^{34}$, His, Met or Gln is substituted for $Ala^{35}$, Phe is substituted for $Leu^{36}$, Gln is substituted for $Ala^{38}$, Asp is substituted for $Asn^{39}$, Phe is substituted for $Val^{41}$, Ser is substituted for $Glu^{42}$, Ser is substituted for $Arg^{44}$, Asn is substituted for $Asp^{45}$, Ser is substituted for $Gln^{47}$, Leu is substituted for $Val^{49}$, Ala, Ser or Leu is substituted for $Cys^{69}$, $Thr^{72}$ or $His^{73}$ is replaced by other amino acids (Japanese Unexamined Patent Publication No. 62-48632), $Leu^{94}$ is deleted or replaced by Tyr, $Ser^{95}$ is deleted, Ala, Ser or Leu is substituted for $Cys^{101}$, or Ala, Ile or Pro is substituted for $Gly^{122}$; a modified polypeptide wherein SerPheValGlnGly is added to the amino terminus; modified polypeptides wherein some amino acids at the amino terminus are replaced by other amino acids having a higher basicity (Japanese Unexamined Patent Publication No. 62-26226); a modified polypeptide wherein $Val^{1}$ to $Ala^{18}$ is replaced by HisSerThrLeuLysProAlaAlaHisLeuIleGly; a modified polypeptide wherein $Ala^{33}$ to $Val^{49}$ is replaced by AspArgAla-PheLeuGlnAspGlyPheSerLeuSerAsnAsnSerLeuLeu; a modified polypeptide wherein $Val^{1}$ to $Thr^{17}$ is replaced by SerPheValGlnGlyGluGluSerAsnAspLysIle; and a combination of these modified polypeptides.

Further, to facilitate the extracellular secretion, the amino acid at the amino terminus of a mature protein to be fused at the carboxyl terminus of the signal peptide is preferably a neutral or hydrophobic amino acid, most preferably Ser. Therefore, to ensure that various modified TNF as above are efficiently secreted out of the cell, it is preferable to effect a modification of basic modified polypeptides wherein 2, 3 or 4 amino acids are deleted at the amino terminus in the human-TNF protein shown in Fig. 1, to the extent that the antitumor activity is not lost or enhanced, that is, to the extent of the modification in the region other than the amino terminus as explained with reference to the above modified human TNF.

As a gene which contains the DNA sequence having a promoter function for controlling expression of the polypeptide having antitumor activity, and the DNA sequence coding for the signal peptide, there may be mentioned an E. coli β-lactamase gene, E. coli alkaline phosphatase gene, E. coli lipoprotein gene, Bacillus subtilis penicillinase gene, Bacillus subtilis protease gene, yeast α-factor gene, alkalophilic Bacillus No. 170 penicillinase gene, alkalophilic Aeromonas No. 212 xylanase gene, alkalophilic Bacillus No. N-4 cellulase gene, alkalophilic Bacillus No. 1139 and the like. Preferably, the alkalophilic Bacillus No. 170 penicillinase gene is used. The promoter sequences in the above genes may be independently combined with the signal peptide sequence in other genes. A gene wherein an appropriate promoter sequence, an appropriate signal peptide sequence, and a DNA sequence coding for the polypeptide having an antitumor activity are linked, in this order, is most preferable.

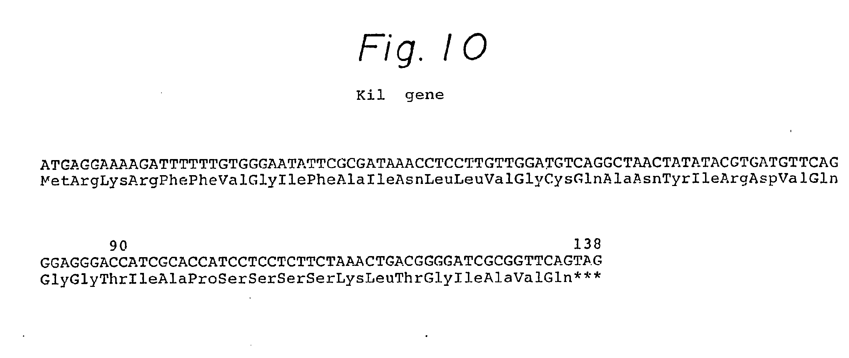

As the DNA sequence practically imparting a function for facilitating the extracellular secretion to a host cell, the DNA sequence coding for the amino acid sequence shown in Fig. 10 is preferable. A typical example thereof is Kil gene from pMB9 plasmid. Other genes coding for the same protein also may be used.

There may be mentioned as a DNA sequence having a promoter function for controlling expression of the DNA sequence practically imparting the function for facilitating the extracellular secretion to the host cell, tryprophan operon promoter (trp promoter), lactose operon promoter (lac promoter), tac promoter, $P_L$ promoter, lpp promoter, Ex promoter stemmed from alkalophilic Bacillus No. 170 and the like. The Ex promoter stemmed from alkalophilic Bacillus No. 170 is particularly preferable, as it has the property of exerting the function thereof at a later stage of logarithmic growth phase of the host cell.

The plasmid according to the present invention comprises the DNA region which expressed the polypeptide having an antitumor activity and the DNA region which has the function of extracellular secretion of the polypeptide having an antitumor activity.

The most preferable DNA region to express the antitumor-active polypeptide is a plasmid wherein an appropriate promoter segment for controlling the expression of the polypeptide having an antitumor activity, an appropriate signal peptide segment, and a DNA segment coding for the polypeptide having an antitumor activity are linked, in this order. Particularly preferably, the appropriate signal peptide sequence and the DNA segment coding for the polypeptide having antitumor activity are linked so that reading frames thereof are engaged with each other.

The DNA region having the function of extracellular secretion of the antitumor-active polypeptide comprises the above DNA sequence practically imparting the function of facilitating extracellular secretion to the host cell, and the sequence having the promoter function for controlling the expression of said DNA sequence.

The antitumor-active polypeptide secreted from the recombinant microbial cell transformed with said plasmid has an homogenity and activity higher than those of the antitumor-active polypeptide taken up from a recombinant microbial cell transformed with an intracellular accumulation type of a plasmid.

Examples of the extracellular secretion expression type of the plasmid are pEXTNF1, pEXTNF3 and pEXTNF4.

15 02 89    0317649

These plasmids have an advantage of an efficient secretion, because the maturation type of the antitumor-active polypeptide can be easily separated, upon secretion, from the signal peptide fused to the polypeptide at the end of the signal peptide.

In particular, pEXTNF3 has Ser at the amino terminus of the polypeptide having an antitumor activity, and thus the secretion efficiency thereof is extremely high.

The above plasmid is introduced into an appropriate host cell in a customary manner to obtain the transformed microbial cell. As the host cell, microorganism belonging to the genus Escherichia may be conveniently used. As the host cell, there may be used the above Escherichia coli strain HB101, E. coli strain C600 (ATCC23724), E. coli strain C600 r⁻m⁻ (ATCC33525), E. coli strain x1776 (ATCC31244), E. coli strain LE392 (ATCC33572), etc., and the microorganisms usually used in this field. Of these microorganisms, E. coli strain HB101 and E. coli strain C600 r⁻m⁻ are particularly preferable.

BRIEF DESCRIPTION OF THE DRAWINGS

Figs. 1 and 2 illustrate base sequences of the human TNF gene and chemically synthesized oligonucleotide, respectively;

Figs. 3 to 5 illustrate a process for a construction of plasmids pTNF1BR, pTNF2N and pTNF3 containing a part of the human TNF gene, respectively;

Fig. 6 illustrates a process for a construction of plasmid pTNF401NN which can express the human TNF gene;

Fig. 7 illustrates a process for a construction of expression plasmid vector pAA41;

Fig. 8 illustrates a process for a construction of plasmid pTNF401A which can highly efficiently express the human TNF gene;

Fig. 9 illustrates a DNA base sequence of a promoter region and signal peptide region of

alkalophilic <u>Bacillus</u> No. 170 penicillinase gene, and the amino acid sequence corresponding to the signal peptide region;

Fig. 10 illustrates a DNA base sequence of a Kil gene present in plasmid pMB9;

Fig. 11 illustrates a DNA base sequence of an Ex promoter region present in chromosomal DNA of alkalophilic <u>Bacillus</u> No. 170;

Fig. 12 illustrates a process for isolation and purification of plasmid DNA from <u>E. coli</u> cells;

Fig. 13 illustrates a process for cloning penicillinase gene of alkalophilic <u>Bacillus</u> No. 170;

Figs. 14, 15 and 16 illustrate a process for the construction of plasmids pEAP3, pEAP6, pEAP7, pEAP7ΔH, pEAP7ΔCCH, pEAP7ΔCH and pEAP8, all containing a DNA region carrying information relating to a production by an extracellular secretion;

Fig. 17 illustrates a process for construction of plasmid pCM71 containing a chloramphenicol acetyl transferase structural gene;

Figs. 18 and 19 illustrate a process for construction of plasmids pPS1, pPS1ΔH, and p329PS, all containing a promoter region and signal peptide region of penicillinase gene;

Fig. 20 illustrates a process for a construction of plasmid pEXTNF1 which can express an extracellular secretion of the antitumor-active polypeptide gene;

Fig. 21 illustrates a result of an activity measurement of the antitumor-active polypeptide contained in a culture supernatant;

Figs. 22 and 23 illustrate a process for a construction of plasmids pEXTNF3 and pEXTNF4 which can express an extracellular secretion of the antitumor-active polypeptide gene, respectively;

Fig. 24 illustrates a result of an activity measurement of the antitumor-active polypeptide contained in a culture supernatant;

Fig. 25 illustrates a result of a confirmation of a secretion of the antitumor-active polypeptide by a western blot technique;

Fig. 26 shows a tumor-carrying mouse before and after administration of the antitumor-active polypeptide; and

Fig. 27 is a graph showing a change of relative weight of a tumor with time, when the antitumor-active polypeptide is administrated to the tumor-carrying mouse.

BEST MODE OF CARRYING OUT THE INVENTION

[Cloning of antitumor-active polypeptide gene]

The human TNF gene can be designed by selecting an appropriate codon from codons designating amino acids composing the human TNF protein [D. Pennica, et al., supra] and can be obtained by chemical synthesis. When designing the human TNF gene, it is preferable to select the codon most suitable for the host cell used, and to provide cleavage sites of appropriate restriction enzymes at appropriate positions to facilitate subsequent cloning and modification of the gene. The DNA sequence coding for the human TNF protein preferably contains a translation initiation codon (ATG) upstream so that the reading frame thereof is engaged, and a translation termination codon (TGA, TAG, or TAA) downstream so that the reading frame thereof is engaged. Particularly preferably, two or more translation termination codons as above are linked in tandem to enhance the expression efficiency. Further, cloning of the human TNF gene in an appropriate vector can be performed by using cleavage sites of a restriction enzyme which works at the upstream and downstream positions thereof. An example of the base sequence of such a human TNF gene is shown in Fig. 1.

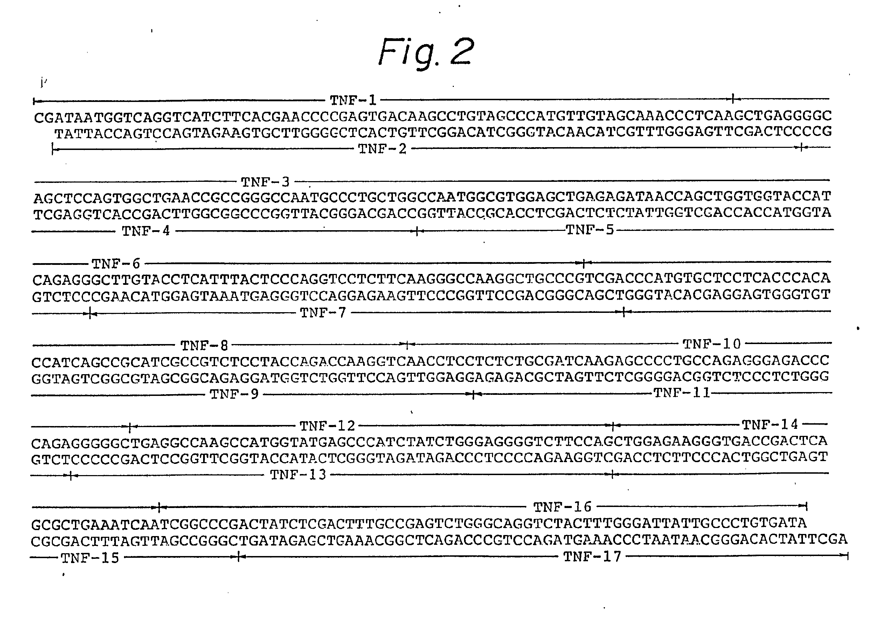

To obtain the human TNF gene as designed above, it is preferable to divides each of upper and lower chains into oligonucleotides as shown in Fig. 2, and chemically

synthesize and then anneal and ligate the oligonucleotides to each other. A process for synthesizing each oligonucleotide includes diester process [H.G. Khorane, "Some Recent Development in Chemistry of Phosphate Esters of Biological Interest", John Wiley and Sons, Inc., New York (1961)], triester process [R.L. Letsinger, et al., J. Am. Chem. Soc., 89, 4801 (1967)] and phosphite process [M.D. Matteucci, et al, Tetrahedron Lett., 21, 719 (1980)]. Because of a synthesizing time, yield, simplicity of operation and so on, synthesizing by the phosphite process using an automatic DNA synthesizer is preferable. The synthesized oligonucleotide may be purified by gel filtration, ion exchange chromatography, gel electrophoresis, and high performance liquid chromatography using a reversed phase column, which are used alone or in combination.

The oligonucleotides thus obtained are phosphated at 5'-terminal hydroxy groups with, for example, T4-polynucleotide kinase, annealed, and then linked with each other using, for example, T4-DNA ligase. A preferable process for construction of human TNF gene by linking synthesized oligonucleotides is a process wherein the oligonucleotides are linked into blocks, the blocks are cloned in a vector such as pBR322 [F. Bolivar, et al., Gene, 2, 95 (1977)], and then DNA fragments in blocks are linked to each other. As the plasmids containing DNA fragments of the blocks composing a human TNF gene, pTNF1BR, pTNF2N or pTNF3 are preferably used.

The cloned DNA fragments of the blocks composing the human TNF gene can be linked to each other, and then connected downstream to an appropriate promoter and SD (Shine-Dalgarno) sequence to form an expression-type gene. The promoters which may be used include tryprophan operon promoter (trp promoter), lactose operon promoter, (lac promoter), tac promoter, $P_L$

promoter, lpp promoter, and the like. The trp promoter is preferable.

As the plasmid having the trp promoter, pYS31N or pAA41 is preferably used. To enhance the expression efficiency, it is possible to provide, downstream of the human TNF gene, a terminater which efficiently works in _Escherichia coli_. The terminaters include lpp terminater, trp terminater and so on, and the trpA terminater is preferable. As a plasmid containing the trpA terminater, pAA41 is preferably used. The expression-type gene of the human TNF can be cloned in, for example, the vector stemmed from pBR322, to form an expression-type plasmid. As the plasmid which can express the human TNF gene, preferably pTNF401NN or pTNF401A is used.

The resulting plasmid which can express the human TNF gene is cleaved with an appropriate enzyme, a particular portion of the human TNF gene is removed, and then the gene is repaired with a synthetic nucleotide having an appropriate base sequence. Using the above technique, it is possible to construct plasmids containing DNA regions coding for antitumor-active polypeptides wherein at least one of any amino acid in the human TNF protein is substituted with another amino acid(s) or deleted therefrom, or other amino acid(s) is (are) added thereto.

[Cloning of penicillinase gene of alkalophilic _Bacillus_ No. 170]

Alkalophilic _Bacillus_ No. 170 (FERM BP-467) is cultivated under an appropriate condition, for example at 30°C with shaking, and the cultivated cells are collected by centrifugation. The cells thus prepared are subjected to a known extraction method such as a phenol method to obtain chromosomal DNA.

The DNA thus obtained is partially cleaved with an appropriate restriction enzyme, such as Eco RI, and the DNA fragment is inserted into the Eco RI restriction site of an appropriate plasmid vector such as plasmid pMB9 to obtain a recombinant plasmid containing the chromosomal DNA of alkalophilic Bacillus No. 170.  The recombinant plasmid is inserted into, for example, E. coli HB101 (ATCC33694), in a known manner, for example, by a $CaCl_2$ method [M.V. Norgard et al., Gene, 3, 279 (1978)], and the resulting transformants are screened for ampicillin and tetracycline resistance to obtain a recombinant plasmid containing the penicillinase gene of alkalophilic Bacillus No. 170 and a DNA region carrying information responsible for the extracellular secretion of penicillinase, for example, plasmid pEAP1.

The DNA base sequence of the resulting recombinant plasmid is determined, for example, by the Maxam-Gilbert method [A.M. Maxam, et al., Methods Enzymol., 65, 499 (1980)] or a dideoxy chain termination method using M13 phage [F. Sanger, Science, 214, 1205 (1981)].

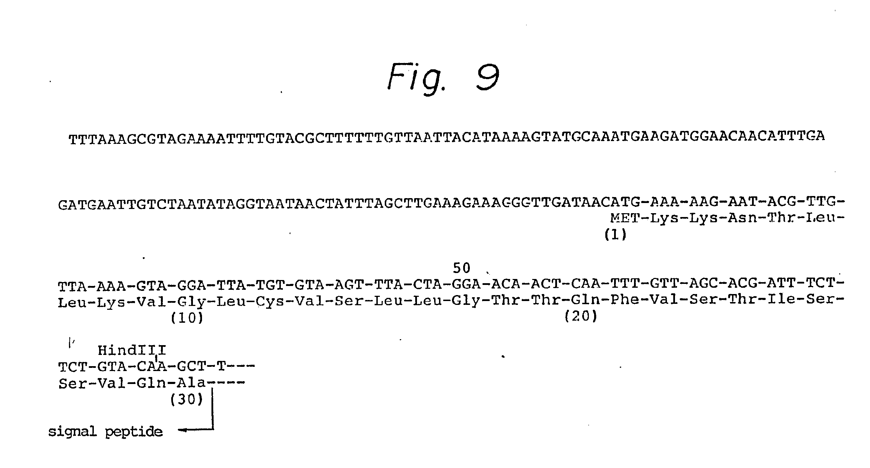

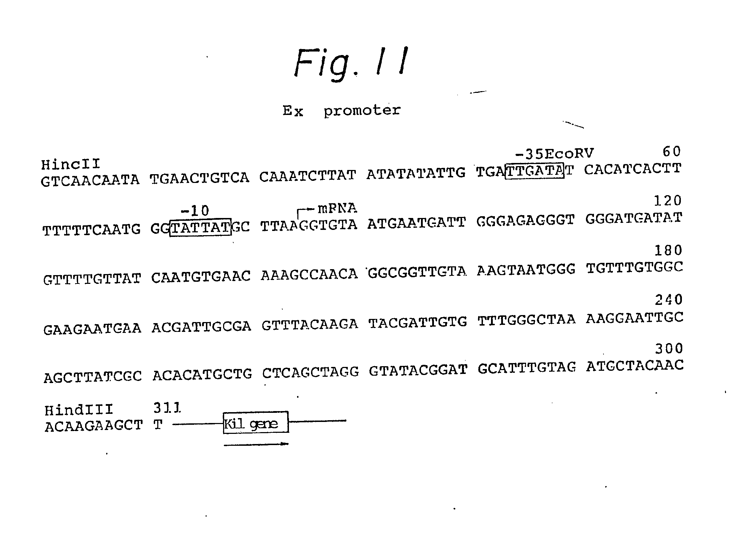

The plasmid thus obtained is then sequenced according to, for example, the above-mentioned Maxam-Gilbert method or a dideoxy chain termination, to clarify the structures of the penicillinase gene promoter region, signal peptide region, mature peptide region, and Ex promoter region responsible for the extracellular secretion of penicillinase and derived from alkalophilic Bacillus No. 170, as well as a Kil gene derived from plasmid pMB9.  Figure 9 represents a DNA base sequence of the promoter region and signal peptide region of the penicillinase gene of the alkalophilic Bacillus No. 170.  Further, Figure 10 shows the DNA base sequence of Kil gene stemmed from plasmid pMB9. Figure 11 shows the DNA base sequence of the Ex promoter stemmed from the alkalophilic Bacillus No. 170.  In the above figure, amino acid sequences corresponding to the signal peptide region and Kil gene are also shown.

Starting from the recombinant plasmid containing a penicillinase gene and a DNA region carrying information responsible for the extracellular secretion of penicillinase, recombinant plasmids containing an entire DNA region carrying information responsible for the extracellular secretion, which region comprises the Ex promoter region derived from alkalophilic <u>Bacillus</u> No. 170 and the Kil gene derived from plasmid pMB9 as well as a part of or the entire penicillinase region are obtained, according to a method using spontaneous deletion or an artificial method using modification by enzymes such as S1 nuclease, DNA polymerase, and the like, and synthetic oligonucleotide. Such plasmids preferably include plasmids pEAP3, pEAP6, pEAP7, pEAP7ΔH, pEAP7ΔCCH, pEAP7ΔCH, and pEAP8.

Moreover, the above-mentioned recombinant plasmid containing a penicillinase gene and a DNA region carrying information responsible for the extracellular secretion of penicillinase is digested with an appropriate restriction enzyme to obtain a DNA fragment containing a promoter region and signal peptide region of penicillinase gene. This DNA fragment is cloned, if necessary via a synthetic oligonucleotide linker, into an appropriate plasmid vector, for example, a hybrid plasmid pCM71 derived from plasmids pCM1 [T.J. Close and R. Rodriguez, Gene, 20, 305, (1982)], and pCM7 [T.J. Close and R. Rodriguez, Gene, 20, 305, (1982)], to obtain a plasmid containing a promoter region and a signal peptide region of the penicillinase gene. As the plasmid containing a promoter region and signal peptide region of the penicillinase gene, plasmids pPS1, pPS1ΔH and p329PS are preferably used.

[Construction of plasmid which makes possible the extracellular secretion of antitumor-active polypeptide gene]

The above-prepared plasmid containing the DNA region coding for the antitumor-active polypeptide is digested with appropriate restriction enzymes to obtain a DNA fragment containing a DNA region coding for the antitumor-active polypeptide.

The DNA fragment then may be inserted into a plasmid containing an appropriate promoter and signal peptide regions to obtain a plasmid wherein the DNA region coding for the antitumor-active polypeptide is linked to the appropriate promoter and signal peptide regions.

As a gene containing such a promoter region and signal peptide region, an E. coli β-lactamase gene, E. coli alkaline phosphatase gene, E. coli lipoprotein gene, Bacillus subtilis penicillinase gene, Bacillus subtilis protease gene, yeast α-factor gene, alkalophilic Bacillus No. 170 penicillinase gene, alkalophilic Aeromonas No. 212 xylanase gene, alkalophilic Bacillus No. N-4 cellulase gene, alkalophilic Bacillus No. 1139 and the like are used. Preferably, an alkalophilic Bacillus No. 170 penicillinase gene is used. The promoter regions in the above genes may be independently combined with the signal peptide regions in the other genes. A plasmid wherein an appropriate promoter region, an appropriate signal peptide region, and the DNA region coding for the antitumor-active polypeptide are linked, in this order, is most preferable. Particularly preferably, the appropriate signal peptide region and the DNA region coding for the antitumor-active polypeptide are linked so that the reading frames thereof are engaged with each other, using a process wherein various enzymes or synthetic oligonucleotides are used.

Further, by combining the various plasmids described above, a plasmid containing both a DNA region comprising an appropriate promoter region, signal peptide region, and the DNA region coding the antitumor-active polypeptide linked downstream from the signal peptide region, and a DNA region carrying information responsible for the extracellular secretion production and comprising an Ex promoter region derived

0317649

from alkalophilic Bacillus No. 170 and Kil gene derived from pMB9 plasmid is obtained. The above plasmid is easily obtained by inserting a DNA fragment containing the DNA region coding for the antitumor-active polypeptide directly into a plasmid containing an appropriate promoter region-signal peptide region and information responsible for the extracellular secretion production. The use of this plasmid makes possible the extracellular secretion of the antitumor-active polypeptide. As the extracellular secretion type plasmid, preferably plasmids pEXTNF1, pEXTNF3 or pEXTNF4 is used.

In the present invention, the appropriate promoter region, signal peptide region, DNA region coding for the antitumor-active polypeptide, Ex promoter region, and Kil gene can produce DNA regions having biological functions equivalent thereto, i.e., a replacement of one or more nucleotides, deletion of one or more nucleotides, insertion of one or more nucleotides, or inversion of a nucleotide sequence, or other mutant polypeptide.

As such a host, microorganisms belonging to the genus Escherichia may be advantageously used. As the host microorganisms customarily used in this field such as E. coli HB101, E. coli C600(ATCC23724), E. coli C600r⁻m⁻ (ATCC33525), E. coli X1776 (ATCC31244), E. coli LE392 (ATCC33572), and the like are preferably used. E. coli HB101 and E. coli C600 r⁻m⁻ are particularly preferable.

The transformant thus obtained is cultivated in a manner known per se. The culture medium which may be used is any medium suitable for the production of the antitumor-active polypeptide and for the growth of the host microorganism. For example, M9 medium [T. Maniatis et al., Molecular Cloning P440, (Cold Spring Harbor Laboratory, New York, 1982)], LB medium [T. Maniatis et al., Molecular Cloning, P440, (Cold Spring Harbor Laboratory, New York, 1982)], BPB medium (Difco), Nutrient Agar medium or the like is used as a basal medium. In addition to a carbon source and nitrogen source, if necessary, nutrients such as amino acids, vitamins or the like may be added to the basal medium. Moreover, an appropriate amount of antibiotics may be added to the medium to stabilize the expression type plasmid in the host cells.

The cultivation is carried out under conditions of the pH, temperature, and oxygen supply level preferable for the recombinant microorganism. When the recombinant microorganism containing the intracellular expression type plasmid is cultivated, the cultivation is preferably continued in a signal medium from the time when the microorganism is grown to reach the maximum cell amount, i.e., a late logarithmic phase, to the time when the antitumor-active polypeptide is formed and accumulated in the medium. For example, in the case of the microorganism belonging to the genus _Escherichia_, a time of about 12 to 48 hours is needed from the time when the cell amount reaches a maximum to the time when the formation and accumulation of the antitumor-active polypeptide in the medium ceases. The pH condition does not particularly affect the result, but the pH value is preferably 5 to 8, more preferably 7.2.

[Evaluation of activity of the antitumor-active polypeptide]

The recombinant microorganism containing the plasmid which makes possible the extracellular secretion is cultivated, and then the microorganism is removed, for example, by centrifugation. The activity of the resulting culture supernatant containing the antitumor-active polypeptide is evaluated directly or after concentration by a customary process therefor. The evaluation of the activity can be conducted by a method for a determination of _in vivo_ activity wherein a necrosis effect is observed on a Meth A sarcoma

- 19 -

0317649

implanted to a mouse (Carswell, et al., supra), a method for a determination of in vitro activity wherein cytotoxicity to a mouse L cell is observed [Ruff, J. Immunol., 126, 235 (1981)] or the like.

[Purification of antitumor-active polypeptide]

Purification of the antitumor-active polypeptide from the culture supernatant of the recombinant microorganism containing the plasmid which makes possible the extracellular secretion can be carried out by a known conventional process for separating and purifying proteins. However, an affinity column chromatography using an antibody to the antitumor-active polypeptide is preferable. An affinity column chromatography using a mouse monoclonal antibody to the antitumor-active polypeptide is particularly preferable. For the resulting purified polypeptide having an antitumor activity, the secretion of the antitumor-active polypeptide from which the signal peptide has been correctly cleaved may be confirmed by an analysis of a molecular weight with SDS-polyacrylamide gel electrophoresis [U.K. Laemmli, Nature, 227, 680 (1970)], an analysis of an amino acid sequence at the amino terminus, and an analysis of a composition of amino acids. Further, the resulting human TNF protein and the purified polypeptide having an antitumor activity may be used to examine carcinostatic activity in vivo, as mentioned.

The antitumor-active polypeptide secreted from the recombinant microorganism transformed with the extracellular secretion-type plasmid according to the present invention, particularly pEXTNF1, pEXTNF3 or pEXTNF4, more particularly pEXTNF3, has a greater homogenity and has a higher antitumor-activity than the antitumor-active polypeptide taken from a recombinant microorganism transformed with an intracellular accumulating type plasmid.

EXAMPLE

The present invention will now be further illustrated by, but is by no means limited to, the following examples.

Example 1   (Design of human TNF gene)

The human TNF gene having the base sequence shown in Fig. 1 was designed.  As the basis for design, the base sequence of the structural gene portion of TNF precursor cDNA reported by Pennica et al [D. Pennica, et al., Nature, 312, 724 (1984)] was used.  Cleavage sites for appropriate restriction enzymes were provided at appropriate positions, a translation initiation codon (ATG) was provided at the 5'-side and two translation termination codons (TGA and TAA) were provided at the 3'-side.  A cleavage site with the restriction enzyme ClaI was provided upstream of the 5'-side translation initiation codon to make a link to a promoter possible while maintaining an appropriate condition between an SD sequence and the translation initiation codon.  Further, a cleavage site with the restriction enzyme HindIII was provided downstream of the 3'-side translation termination codon to facilitate the linkage to a vector plasmid.

Example 2   (Chemical synthesis of oligonucleotide)

The human TNF gene designed in Example 1 was prepared by separately synthesizing 17 oligonucleotides as shown in Fig. 2.  The oligonucleotides were synthesized by a phosphite method using a DNA synthesizer (Applied Biosystems, Model 380A).  The synthetic oligonucleotides were purified according to a manual disclosure by Applied Biosystems.  That is, an aqueous ammonium solution containing synthetic oligonucleotides was maintained at 55°C overnight to remove protecting groups of the DNA bases, and the solution was subjected to gel filtration using Sephadex G-50 fine gel (Pharmacia) to isolate synthetic oligonucleotides with a high molecular weight.  Then, the isolated products were subjected to polyacrylamide gel electrophoresis (gel concentration 20%) with 7 M urea, and migration patterns were observed with a UV shadowing procedure. The portions having the desired size were cut off, the resulting polyacrylamide gel fragments were finely divided, and after adding 2-5 ml of elute buffer [500 mM $NH_4OAc$-1 mM EDTA-0.1% SDS (pH 7.5)], shaken at 37°C overnight, and by centrifugation, aqueous phases containing the desired DNAs were recovered. Finally, solutions containing synthetic oligonucleotides were applied on a gel filtration column (Sephadex G-50) to obtain purified synthetic oligonucleotides. If necessary, the polyacrylamide gel electrophoresis was repeated to improve the purify of the oligonucleotides.

Example 3 (Cloning of chemically synthesized human TNF gene)

The human TNF gene was separately cloned in the form of three blocks, using 17 synthetic oligonucleotide (TNF-1 to TNF-17) prepared in Example 2.

The synthetic oligonucleotides TNF-2 to TNF-6 (0.1 - 1.0 μg) were separately phosphorylated at 5'-termini thereof, using 5 - 15 units of T4-polynucleotidekinase (E. coli B type, Takara Shuzo). Phosphorylation was carried out at 37°C for 30 minutes in an aqueous solution of 10 - 20 μl of 50 mM Tris-HCl (pH 9.5), 10 mM $MgCl_2$ , 5 mM dithiothreitol and 10 mM ATP. After the reaction was completed, all solutions of the synthetic oligonucleotides were mixed, and then T4-oligonucleo-tidekinase was inactivated and removed by a phenol extraction and an ether extraction. To the mixture of the solutions of the synthetic oligonucleotides, the synthetic oligonucleotides TNF-1 and TNF-7 (0.1 - 1.0 μg) were freshly added, the whole was heated at 90°C for 5 minutes, and then cooled gradually to the room temperature to carry out an annealing. After drying under the reduced pressure, the product was dissolved in 30 μl of an aqueous solution containing 66 mM Tris-HCl (pH 7.6), 6.6 mM $MgCl_2$ , 10 mM

dithiothreitol and 1 mM ATP. After adding 300 units of T4-DNA ligase (Takara Shuzo), ligation was carried out at 11°C for 15 hours. After the reaction was completed, polyacrylamide gel electrophoresis (gel concentration; 5%) was carried out and the migration pattern was observed, using an ethidium bromide staining method. The portion of the band having the desired size (about 220 bp) was cut off, and DNA was recovered from the polyacrylamide gel in accordance with the procedure described in Example 2.

On the other hand, 3 µg of plasmid pBR322 (about 4.4 Kbp) (Bethesda Research Laboratories) for Escherichia coli was dissolved in 30 µl of an aqueous solution containing 10 mM Tris-HCl (pH 7.5), 60 mM NaCl and 7 mM MgCl$_2$. After adding 10 units of the restriction enzyme ClaI (New England Biolabs), a cleavage reaction was carried out at 37°C for 1 hour. After the cleavage with the restriction enzyme ClaI, a phenol extraction, an ether extraction and an ethanol precipitation were performed to recover DNA. The DNA was dissolved in 30 µl of an aqueous solution containing 50 mM Tris-HCl (pH 7.4), 100 mM NaCl and 10 mM MgSO$_4$. After 10 units of the restriction enzyme SalI (Takara Shuzo), a cleavage reaction was carried out at 37°C for 1 hour. After the reaction was completed, agarose gel electrophoresis (gel concentration; 0.8%) was carried out and the cut-off pattern was observed, using an ethidium bromide staining method. The portion of the band corresponding to DNA having about 3.7 Kbp and containing almost all of plasmid pBR322 was cut off, the resulting agarose gel fragment was dissolved in a three times amount (vol/wt) of an aqueous solution containing 8M NaClO$_4$, and a DNA fragment having about 3.7 Kbp (ClaI ←→ SalI) was recovered from the agarose gel by a glass filter method of Chen, et al., [C.W. Chen, et al., Anal. Biochem., 101, 339 (1980)].

The above-mentioned DNA fragment (about 220 bp)

0317649

containing a past of the human TNF gene was phosphorylated at the terminus thereof, as in the above procedure, and then mixed with the aqueous solution of DNA (about 3.7 Kbp) containing almost all of the plasmid pBR322. After an ethanol precipitation, both of the DNA fragments were ligated as in the above procedure.

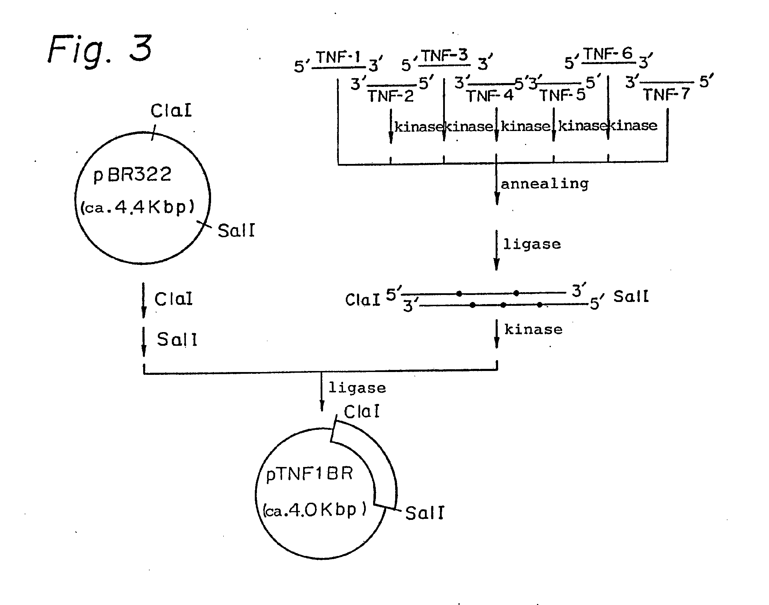

A transformation of <u>Escherichia coli</u> C600r⁻m⁻ was performed by a modified conventional $CaCl_2$ method (the method of M.V. Norgard, et al.). Namely, eighteen-hour culture of <u>Escherichia coli</u> C600r⁻m⁻ was inoculated in 5 ml of an L medium (1% trypton, 0.5% of yeast extract, and 0.5% of NaCl, pH 7.2), and the <u>E. coli</u> was cultivated until a turbidity at 600 nm ($OD_{600}$) reached 0.3. The cultivated cells were washed twice in a cold magnesium buffer [0.1 M NaCl, 5 mM $MgCl_2$ , 5 mM Tris-HCl (pH 7.6 at 0°C)], resuspended in 2 ml of a cold calcium buffer [100 mM $CaCl_2$ , 250 mM KCl, 5 mM $MgCl_2$ 5 mM Tris-HCl (pH 7.6 at 0°C)], and the suspension was allowed to stand for 25 minutes at 0°C. Then, the cells were concentrated in the calcium buffer to a 1/10 volume thereof, and mixed with the above-mentioned ligated DNA aqueous solution at a ratio of 2:1 (vol:vol). The mixture was maintained for 60 minutes at 0°C. After 1 ml of an LBG medium (1% trypton, 0.5% yeast extract, 1% NaCl and 0.08% glucose, pH 7.2) was added, cultivation was carried out at 37°C for one hour with shaking. The cultured broth was inoculated at 100 μl/plate on a selective medium (L medium plate containing 30 μg/ml of ampicillin), and the plates were incubated at 37°C overnight to grow transformants. From the ampicillin resistant colonies, DNA was prepared by a conventional method. An agarose gel electrophoresis confirmed that a desired plasmid pTNF1BR (about 4.0 Kbp) was obtained. Figure 3 shows the process for a construction of the plasmid pTNF1BR.

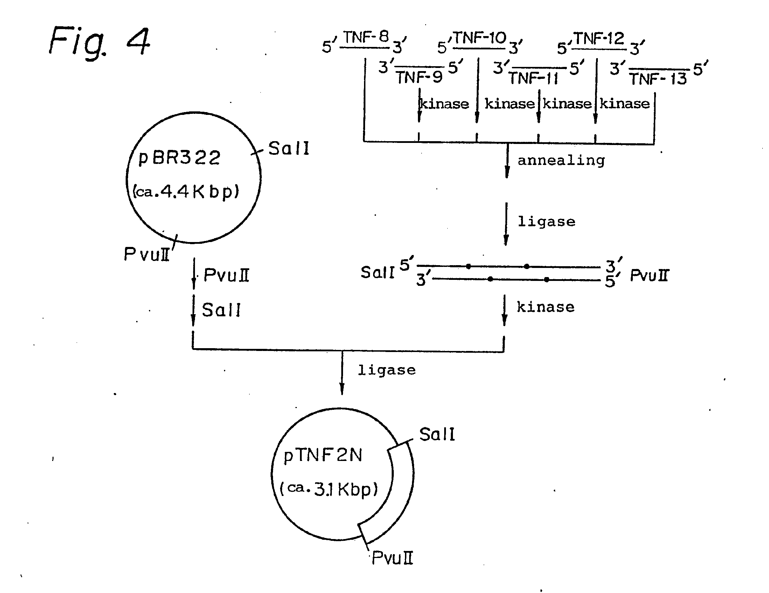

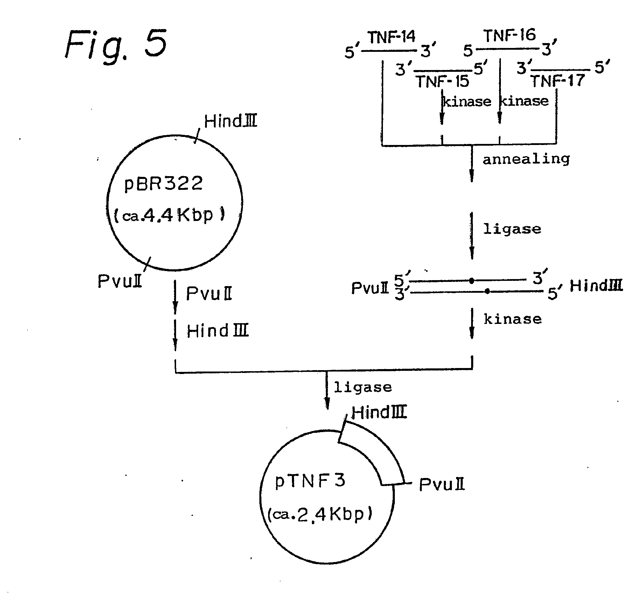

By the above procedure, plasmid pTNF2N (about 3.1 Kbp) and plasmid pTNF3 (about 2.4 Kbp) were prepared from synthetic oligonucleotides TNF-8 to TNF-13 and synthetic oligonucleotides TNF-14 to TNF-17, respectively. Figures 4 and 5 illustrate a process for a construction of plasmid pTNF2N or plasmid pTNF3. By the Maxam-Gilbert method (A.M. Maxam, et al., supra), it was confirmed that the base sequences of portions of the synthetic oligonucleotides used are consistent with those of the design in the resulting plasmids pTNF1BR, pTNF2N and pTNF3 containing parts of the human TNF gene.

Example 4 (Construction of plasmid which can express human TNF gene)

The plasmid pTNF1BR (10 μg) prepared in Example 3 was cleaved with restriction enzymes ClaI and SalI as in Example 3. After polyacrylamide gel electrophoresis (gel concentration: 5%), a DNA fragment (ClaI ←——→ SalI) (about 220 bp) containing a part of the human TNF gene was recovered from the polyacrylamide gel.

Then, the plasmid pTNF2N (10 μg) prepared in Example 3 was dissolved in 100 μl of an aqueous solution containing 10 mM Tris-HCl (pH 7.5), 60 mM NaCl, and 7 mM MgCl$_2$. After adding 40 units of restriction enzyme PvuII (Takara Shuzo), a cleavage reaction was carried out at 37° for 1 hour. After cleavage with a restriction enzyme SalI and polyacrylamide gel electrophoresis (gel concentration: 5%) as in Example 3, a DNA fragment (SalI ←——→ PvuII) (about 170 bp) containing a part of the human TNF gene was recovered from the polyacrylamide gel as in Example 2.

Further, the plasmid pTNF3 (10 μg) prepared in Example 3 also was dissolved in 100 μl of an aqueous solution containing 10 mM Tris-HCl (pH 7.5), 60 mM NaCl and 7 mM MgCl$_2$. After adding 40 units of restriction enzyme PvuII and 40 units of restriction enzyme HindIII (Takara Shuzo), a cleavage reaction was carried out at 37°C for 1 hour. After polyacrylamide gel electrophoresis (gel concentration: 5%), DNA fragment (PvuII ←——→ HindIII) (about 110 bp) containing a part the human TNF gene was recovered from the polyacrylamide gel.

On the other hand, 5 μg of plasmid pYS31N (about 4.7 Kbp) having E. coli trp promoter (described in Japanese Unexamined Patent Publication No. 62-153300) was cleaved with restriction enzymes ClaI and HindIII, and subjected to agarose gel electrophoresis (gel concentration: 0.8%) as above, and a DNA fragment (ClaI ⟷ HindIII) (about 4.7 Kbp) containing the most part of plasmid pYS31N was recovered from the agarose gel.

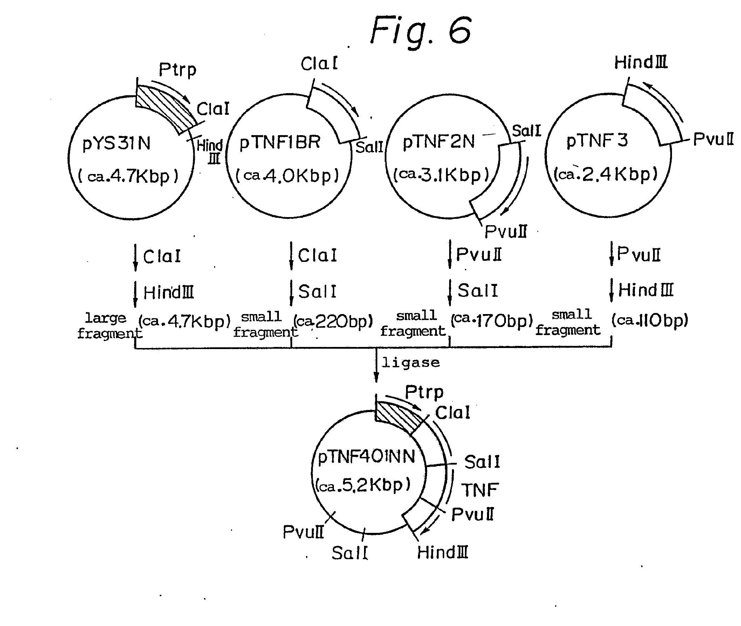

The resulting three DNA fragments (about 220 bp, about 170 bp, and about 110 bp), each containing a part of the human TNF gene, and the DNA fragment (about 4.7 Kbp) containing the most part of plasmid pYS31N were mixed together. After ethanol precipitated, a ligation reaction was carried out with T4-DNA ligase as in Example 3, and after the reaction was completed, a transformation into Escherichia coli C600r⁻m⁻ was performed as in Example 3. Among the transformants, clones containing a desired human TNF gene expression type plasmid pTNF401NN (about 5.5 Kbp) were selected. Figure 6 shows a process for the construction of the above plasmid pTNF401NN.

Further, 5 μg of the above plasmid pYS31N was partially digested with a restriction enzyme PvuII, and then cleaved with a restriction enzyme HindIII as in the above process. After agarose gel electrophoresis (gel concentration: 0.8%), a DNA fragment [PvuII (2) ⟷ HindIII] (about 2.7 Kbp) containing the trp promoter was recovered from the agarose gel.

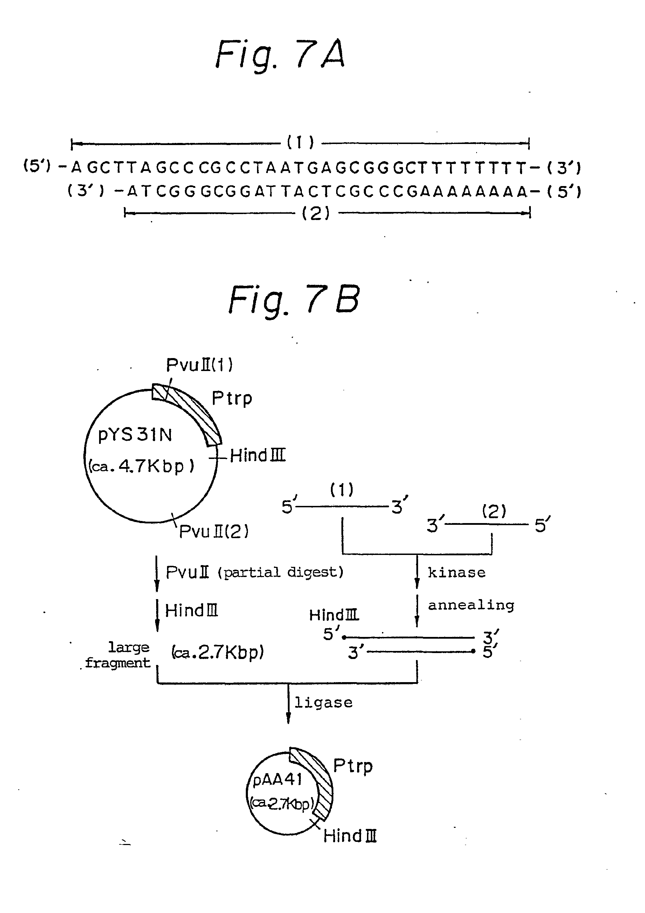

Then, the oligonucleotide having the base sequence shown in Fig. 7A was synthesized and purified as in Example 2, two chains of the resulting synthetic oligonucleotides (0.5 μg each) were phosphorylated at the termini thereof as in Example 3, and after annealing, the product was mixed with a DNA fragment [PvuII ⟷ HindIII] (about 2.7 Kbp) previously obtained. After ethanol precipitation, a ligation reaction with T4-DNA ligase was carried out as in Example 3, and after the reaction was completed, a transformation into Escherichia coli C600r⁻m⁻ was conducted as in Example 3. Among the transformants, clones having a desired plasmid pAA41 (about 2.7 Kbp) were selected. The plasmid is a high copy number and efficient expression vector wherein a region responsible for limiting the number of copies is removed from plasmid pYS31N, and an E. coli trp A terminater is provided downstream of a cloning site downstream of the trp promoter. Figure 7 shows a process for the construction of the plasmid.

. The plasmid pAA41 (2 μg) was cleaved with restriction enzymes ClaI and HindIII as in the above procedure, and after agarose gel electrophoresis (gel concentration: 0.8%), a DNA fragment (ClaI ⟷ HindIII) (about 2.7 Kbp) containing the most part of plasmid pAA41 was recovered from the agarose gel.

The human TNF gene expression plasmid pTNF401NN (5 μg) previously obtained was cleaved with restriction enzymes ClaI and HindIII as above, and after polyacrylamide gel electrophoresis (gel concentration: 5%), a DNA fragment (ClaI ⟷ HindIII) (about 490 bp) containing all of the human TNF gene was recovered from the polyacrylamide gel.

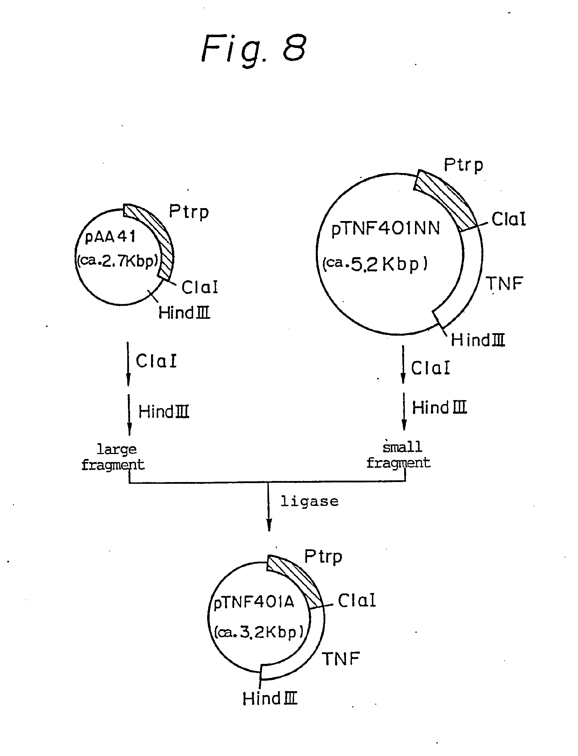

The resulting DNA fragment (about 2.7 Kbp) containing the most part of plasmid pAA41 was mixed with a DNA fragment (about 490 bp) containing all of the human TNF gene, and after ethanol precipitation, a ligation reaction with T4-DNA ligase was carried out as in Example 3. After the reaction was finished, a transformation into Escherichia coli 600r⁻m⁻ was conducted as in Example 3. Among the transformants, clones having a desired plasmid pTNF401A (about 3.2 Kbp) were selected. This plasmid is capable of expressing the human TNF gene more efficiently. Figure 8 shows a process for the construction of the plasmid.

Example 5 (Preparation of chromosomal DNA of alkalophilic Bacillus No. 170)

Alkalophilic Bacillus No. 170 (FERM BP-467) can produce and accumulate penicillinase within cells was cultivated in a medium containing 2.0 g/l glycerol, 5.0 g/l yeast extract, 5.0 g/l polypepton, 1.0 g/l $K_2HPO_4$ and 0.2 g/l $MgSO_4 \cdot 7H_2O$ (adjusted to pH 9.0 with 10 g/l $NaHCO_3$) at 30°C for 15 hours with shaking. The cultivated cells at a late logarithmic phase were collected. Chromosomal DNA was extracted from the cells according to a phenol extraction method to obtain 5 mg of chromosomal DNA.

. Example 6 (Insertion of chromosomal DNA fragment derived from alkalophilic Bacillus No. 170 into vector)

Chromosomal DNA (10 $\mu$g) obtained in Example 5 was partially digested with a restriction enzyme EcoRI at 37°C according to a customary procedure. On the other hand, the plasmid pMB9 resistant to a tetracycline (Bethesda Research Laboratories) was completely digested with the restriction enzyme EcoRI, and the digestion product was heated at 65°C for five minutes, and mixed with the digestion product of chromosomal DNA as above prepared. The mixture was subjected to ligation using T4-DNA ligase at 10°C for 24 hours according to the procedure described in Example 3. After heating at 65°C for 5 minutes, ethanol at twice the volume was added to the reaction mixture to precipitate plasmid DNA containing the inserted chromosomal DNA.

Example 7 (Cloning of penicillinase gene derived from alkalophilic Bacillus No. 170)

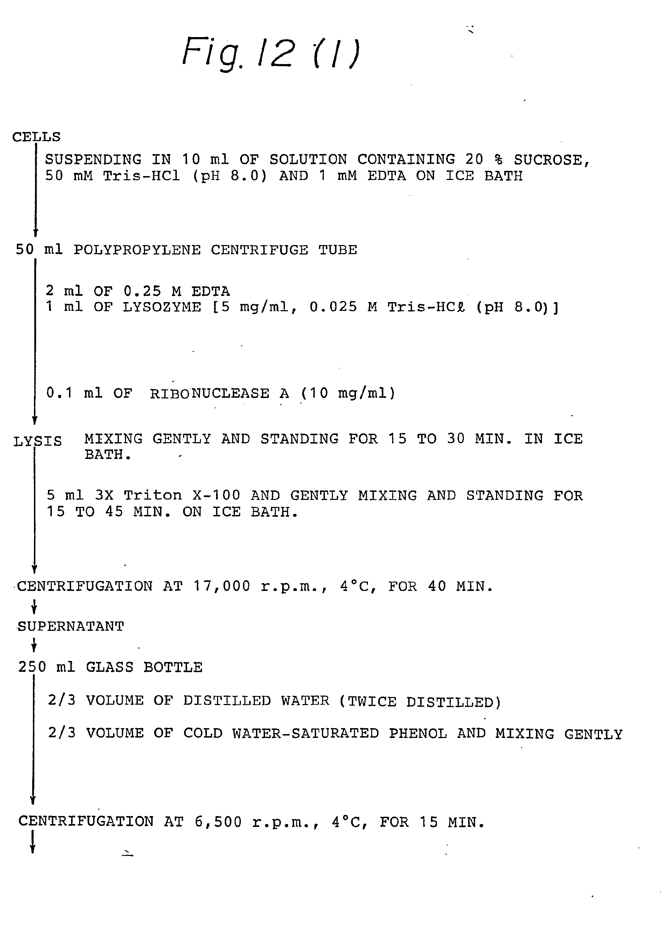

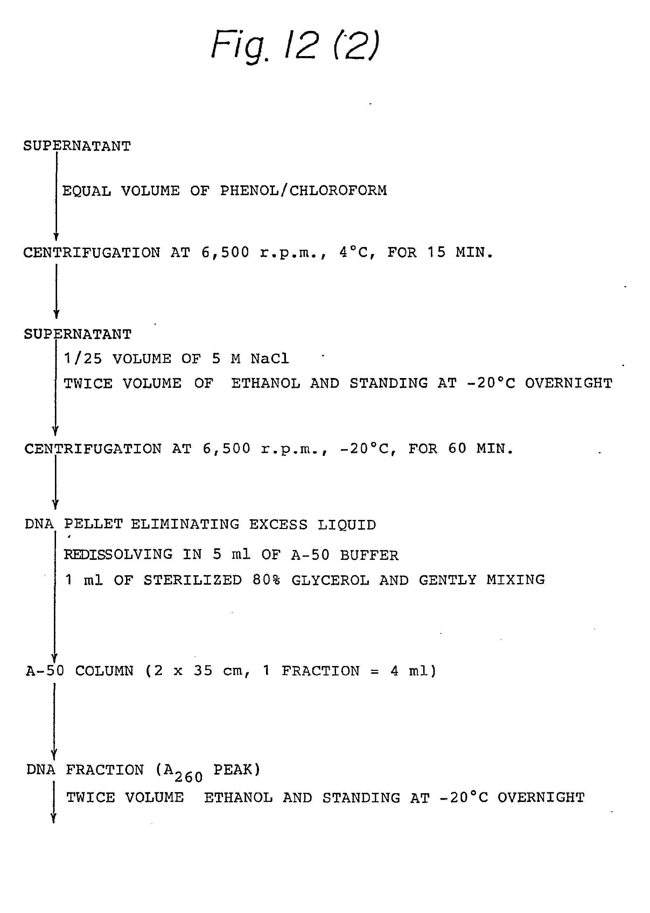

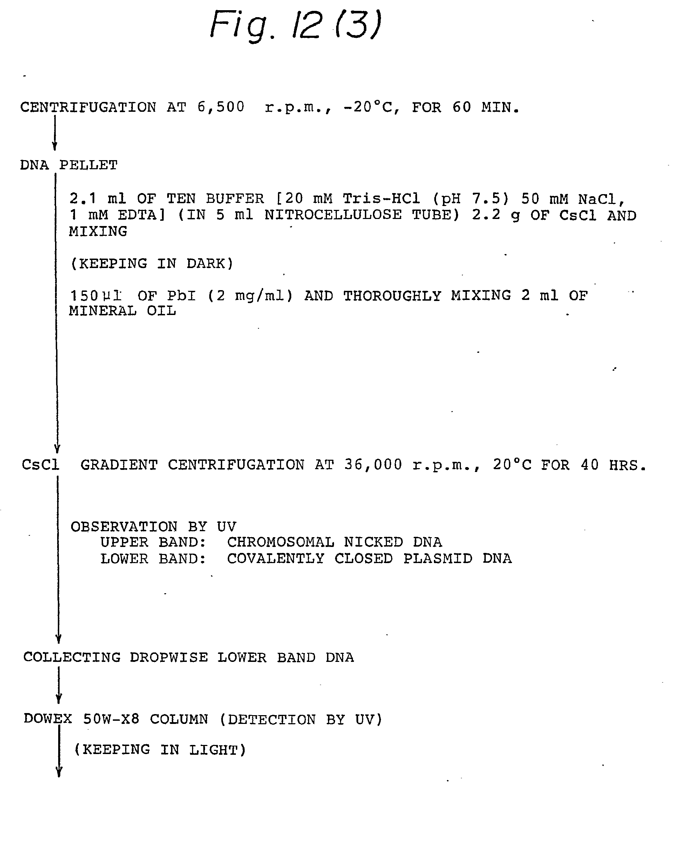

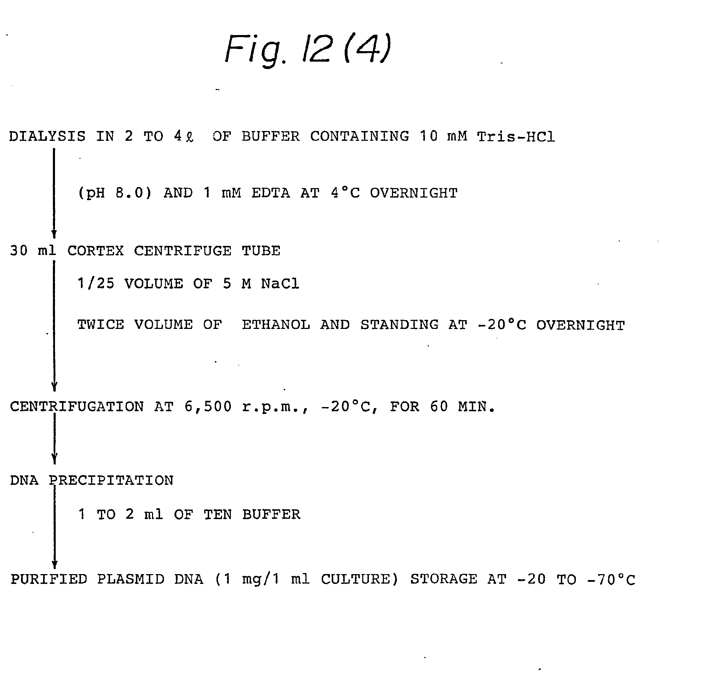

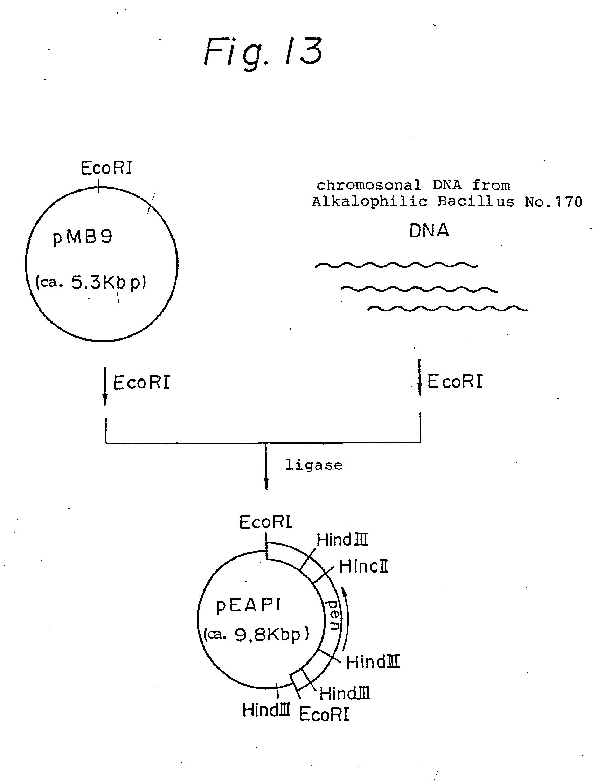

Plasmid containing a chromosomal DNA of alkalophilic Bacillus No. 170, prepared in Example 6, was transformed into E. coli HB101 by a customary $CaCl_2$ method (M.V. Norgard, et al., supra). The transformants were screened for ampicillin resistance and tetracycline resistance, and a clone containing a penicillinase gene of alkalophilic Bacillus No. 170 was selected. After cultivating the selected cells, the cells were treated according to the procedure shown in Fig. 12 to isolate a recombinant plasmid pEAP1 containing a 4.5 Kbp insert comprising penicillinase gene. A restriction-enzyme cleavage-site map for the plasmid was prepared by carrying out cleavages with various restriction enzymes according to a customary procedure. Figure 13 shows a restriction-enzyme cleavage-site map of the plasmid pEAP1.

Example 8 (Determination of DNA base sequence of plasmid containing chromosomal DNA of alkalophilic Bacillus No. 170)

A DNA base sequence of plasmid containing chromosomal DNA of alkalophilic Bacillus No. 170 was determined according to a dideoxy chain termination method (F. Sanger, supra) by M13 phase using an M13 Sequencing kit (Amersham). Figure 9 shows a DNA base sequence of the promoter region and signal peptide region of the penicillinase gene of alkalophilic Bacillus No. 170; Figure 10 shows a DNA base sequence of Kil gene derived from plasmid pMB9; and Figure 11 shows a DNA base sequence of the Ex promoter region derived from chromosomal DNA of alkalophilic Bacillus No. 170.

Example 9 (Construction of various plasmid derivatives containing chromosomal DNA of alkalophilic Bacillus No. 170).

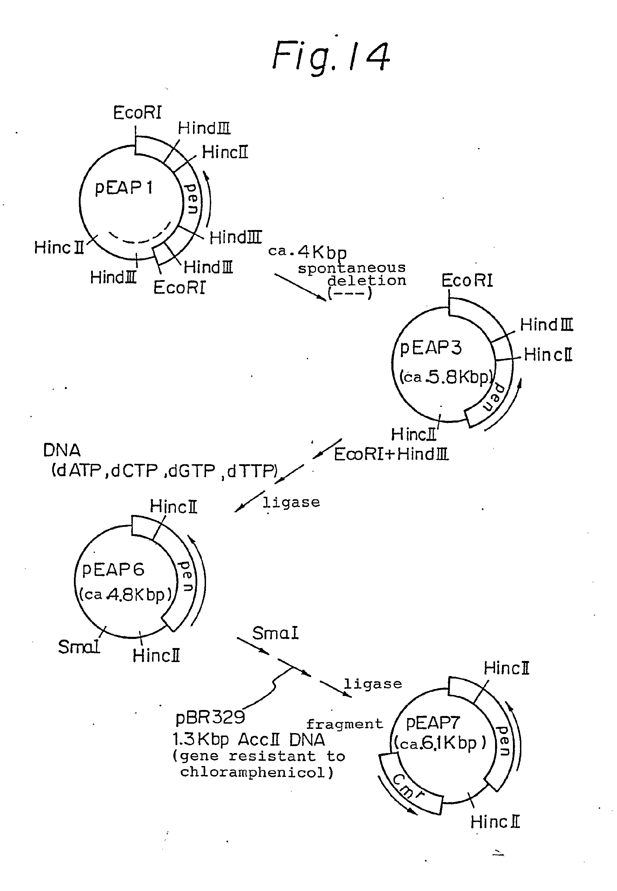

In the course of the passage of Escherichia coli HB101 containing the plasmid pEAP1, obtained in Example 7, a mutant (ampicillin resistant, tetracycline sensitive) exhibiting an enhanced penicillinase activity three times higher than that of pEAP1 was obtained. From the mutant, a plasmid was prepared according to the procedure illustrated in Fig. 12, and thus, plasmid pEAP3 (about 5.8 Kbp) wherein about 4 Kbp of DNA upstream to penicillinase structural gene has been deleted, was obtained. Figure 14 shows a restriction-enzyme cleavage-site map of plasmid pEAP3.

One µg of the plasmid pEAP3 thus obtained was digested with restriction enzymes EcoRI and HindIII at 37°C for 2 hours according to a customary procedure. After adding a DNA polymerage I large fragment in accordance with a customary procedure, a reaction was carried out at a room temperature for 30 minutes to make ends blunt. Then, the ligation of DNA chains was carried out with a T4-DNA ligase at a room temperature for 24 hours as in Example 3. After heating at 65°C for 5 minutes, ethanol at twice the volume was added to the reaction liquid to precipitate and obtain plasmid DNA. The plasmid DNA thus obtained was used to transform E. coli HB101 as in Example 7, and from an ampicillin resistant transformant, plasmid was isolated and purified according to the procedure described in Fig. 12. Thus the plasmid pEAP6 (about 4.8 Kbp) wherein an EcoRI ←→ HindIII DNA fragment (about 1.0 Kbp) had been deleted was obtained. Figure 14 shows a process for the construction of the plasmid pEAP6.

Then, 10 µg of DNA of plasmid pBR329 (about 4.15 Kbp) [L. Covarrubias, et al., Gene, 17, 79, (1982)] was cleaved with a restriction enzyme AccII by a customary procedure. After an agarose gel electrophoresis (gel concentration: 1.5%), an AccII ←→ AccII DNA fragment (about 1.3 Kbp) containing chloramphenicol acetyl transferase (CAT) gene was recovered by an electroelution method [P.J. Greene, et al., Methods in Molecular Biology, Vol. 7, Marcell Dekker, 1978, p87]. On the other hand, the plasmid pEAP6 obtained as described above was cleaved with a restriction enzyme SmaI according to a customary procedure. After mixing with the above-mentioned AccII ←→ AccII DNA fragment, a ligation was carried out using T4-DNA ligase as in Example 3. As described above, transformation of E. coli HB101 was carried out, and among a chloramphenicol and an ampicillin resistant transformant, plasmid was isolated and purified. Thus, the plasmid pEAP7 (about 6.1 Kbp) wherein the CAT gene was inserted into plasmid pEAP6 in the counterclockwise direction as shown in Fig. 14 was obtained. Figure 14 illustrates a process for the construction of the plasmid pEAP7.

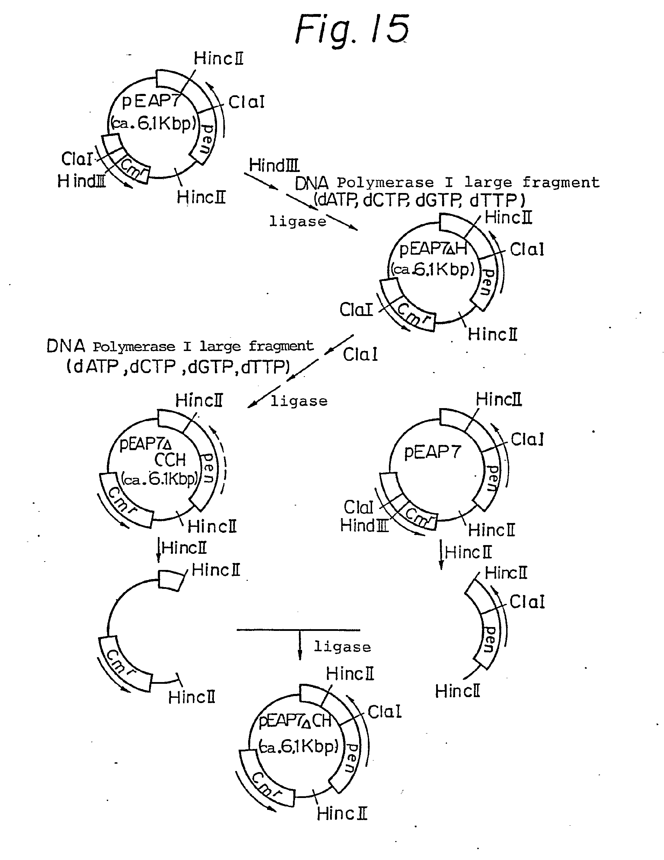

The plasmid pEAP7 thus obtained was cleaved with a restriction enzyme HindIII according to a customary procedure, and a DNA polymerase I large fragment was used to make ends blunt according to a customary procedure. Self ligation was carried out using T4-DNA ligase as in Example 3, to construct a plasmid pEAP7ΔH (about 6.1 Kbp) wherein the HindIII site in plasmid pEAP7 has been deleted. Figure 15 shows a process for the construction of the plasmid pEAP7ΔH.

Then, plasmid pEAP7ΔH was cleaved with a restriction enzyme ClaI according to a customary procedure, and a DNA polymerase I large fragment was used to make ends blunt according to a customary procedure. Ligation was carried out using T4-DNA ligase as in Example 2, and transformation of E. coli HB101 was carried out. Among chloramphenicol resistant and ampicillin sensitive transformant, a plasmid was isolated and purified. Thus, the plasmid pEAP7ΔCCH wherein both of the two ClaI sites present in plasmid pEAP7ΔH have been deleted was obtained. Figure 15 shows a process for the construction of the plasmid pEAP7ΔCCH.

The plasmid pEAP7ΔCCH thus obtained was cleaved with a restriction enzyme HincII according to a customary procedure. After an agarose gel electrophoresis (gel concentration: 0.8%), an HincII ⟷ HincII DNA fragment (about 3.8 Kbp) wherein a portion of the penicillinase gene has been deleted was recovered from the agarose gel by an electroelution method. On the other hand, the above-mentioned plasmid pEAP7 was cleaved with a restriction enzyme HincII in the same manner as described above. After an agarose gel electrophoresis (gel concentration: 0.8%), an HincII ⟷ HincII DNA fragment (about 2.3 Kbp) containing penicillinase gene was recovered.  These two DNA fragments were ligated using T4-DNA ligase as in Example 3, and a transformation of E. coli HB101 was carried out.  Among a chloramphenicol and ampicillin resistant transformant, a plasmid pEAP7ΔCH (about 6.1 Kbp) was obtained.  Figure 15 shows a process for construction of the plasmid pEAPΔ7CH.

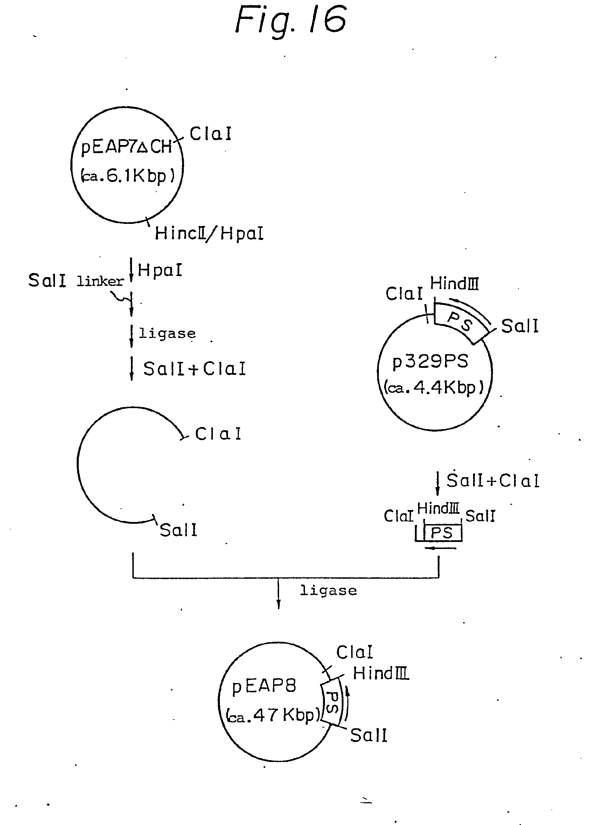

The plasmid pEAP7ΔCH thus obtained was cleaved with a restriction enzyme HpaI according to a customary procedure, and then mixed with a SalI linker phosphorylated at its 5'-end (Takara Shuzo).  Ligation was carried out using T4-DNA ligase as in Example 3.  After ethanol precipitation, the ligation product was cleaved with restriction enzymes ClaI and SalI.  After an agarose gel electrophoresis (gel concentration:  0.8%), a ClaI ←→ SalI DNA fragment (4.5 Kbp) containing a latter half of penicillinase gene, Ex promoter region, and a major portion of vector was recovered by an electroelution method.  On the other hand, the plasmid p329PS obtained in Example 10 described below was similarly cleaved with restriction enzymes ClaI and SalI.  After an polyacrylamide gel electrophoresis (gel concentration:  5%), a SalI ←→ ClaI DNA fragment (about 200 bp) containing a penicillinase gene promoter region and signal peptide region was recovered by an electroelution method.  The two DNA fragments thus obtained were mixed.  After ligation was carried out using T4-DNA ligase as in Example 3, transformation of E. coli HB101 was carried out in the same manner as described above.  From a chloramphenicol resistant transformant, secretion type plasmid vector pEAP8 (4.7 Kbp) was isolated and purified.  Figure 16 illustrates a process for the construction of the plasmid pEAP8.

Escherichia coli HB101 transformed with the pEAP8, i.e., Escherichia coli HB101 [pEAP8] has been deposited since June 10, 1988 in the Fermentation Research Institute, Agency of Industrial Science and Technology, Ministry of International Trade and Industry under the deposition number of FERM BP-1909.

Example 10 (Construction of plasmid containing a promoter region and signal peptide region of penicillinase gene of alkalophilic microorganism)

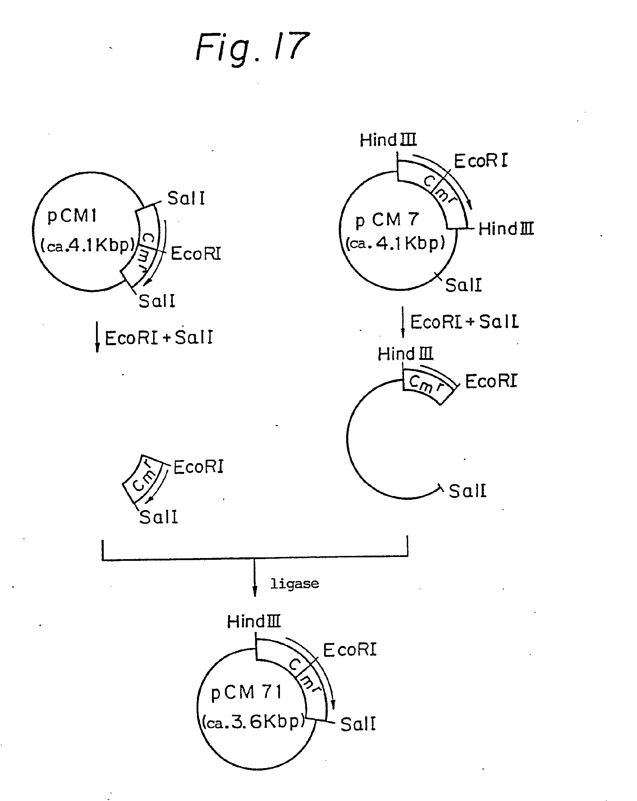

Plasmid pCM1 (about 4.1 Kbp: Pharmacia) containing CAT gene derivative was cleaved with restriction enzymes EcoRI and SalI according to a customary procedure. After an agarose gel electrophoresis (gel concentration: 0.8%), an EcoRI ⟷ SalI DNA fragment (about 0.5 Kbp) containing the latter half of CAT gene was recovered as in Example 9. Further, plasmid pCM7 (about 4.1 Kbp: Pharmacia) containing a CAT gene derivative was cleaved with restriction enzymes EcoRI and SalI as described above. After an agarose gel electrophoresis (gel concentration: 0.8%), an EcoRI ⟷ SalI DNA fragment (about 3.1 Kbp) comprising the first half of the CAT gene and a major portion of vector was recovered. These DNA fragments were mixed and ligated using T4-DNA ligase as in Example 3, and a new plasmid pCM71 (about 3.6 Kbp) containing the CAT gene derivative was constructed as in Example 9. Figure 17 shows a process for the construction of the plasmid pCM71.

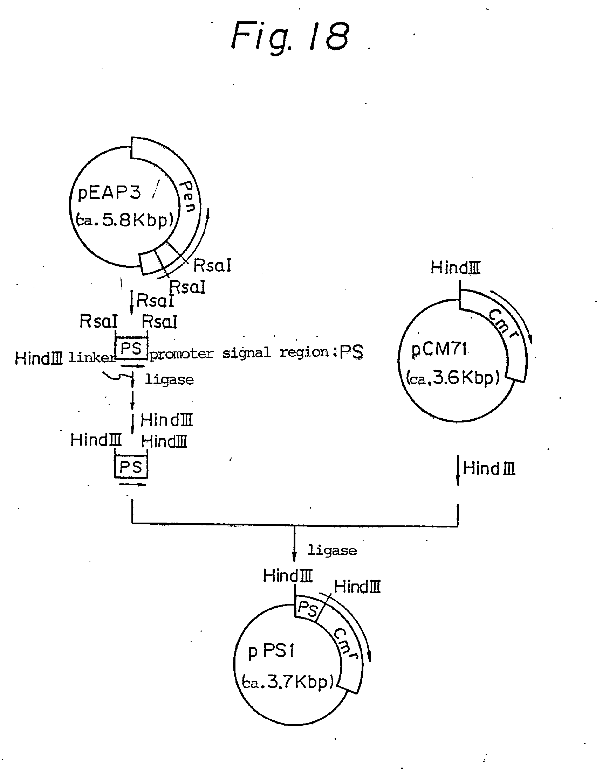

The plasmid pEAP3 constructed in Example 9 was cleaved with a restriction enzyme RsaI as in Example 9. After polyacrylamide gel electrophoresis (gel concentration: 5%), an RsaI ⟷ RsaI DNA fragment (about 200 bp) containing a promoter region and signal peptide region of a penicillinase gene was recovered as in Example 9. This RsaI ⟷ RsaI DNA fragment was mixed with a HindIII linker having a phosphorylated terminus (Takara Shuzo). After ligation was carried out using T4-DNA ligase as in Example 3, the ligation product was cleaved with a restriction enzyme HindIII according to a customary procedure. After polyacrylamide gel electrophoresis (gel concentration: 5%), a HindIII - ended DNA fragment (about 210 bp) containing a promoter region and signal peptide region of the penicillinase gene was recovered as in Example 9. On the other hand, the above plasmid pCM71 was cleaved with a restriction enzyme HindIII according to a customary procedure, and mixed with the above-mentioned HindIII $\longleftrightarrow$ HindIII DNA fragment containing a promoter region and signal peptide region of penicillinase gene. These fragments were ligated using T4-DNA ligase as in Example 3. From an ampicillin and chloramphenical resistant transformant, a plasmid pPS1 (about 3.7 Kbp) was isolated and purified. In this plasmid pPS1, a promoter region and signal peptide region of penicillinase gene was inserted upstream to the CAT gene in the same reading orientation. Figure 18 shows a process for the construction of the plasmid pPS1.

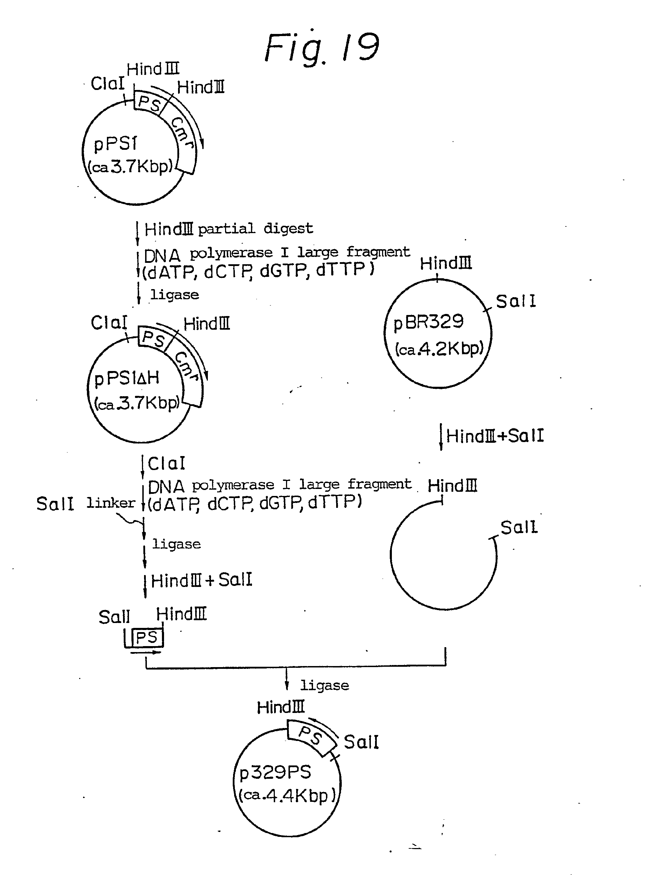

The plasmid pPS1 thus obtained was partially digested with the restriction enzyme HindIII according to a customary procedure. After agarose gel electrophoresis (gel concentration: 0.8%), a HindIII $\longleftrightarrow$ HindIII DNA fragment (about 3.7 Kbp) formed by cleavage of one of two HindIII sites was recovered in the same manner as described above. This HindIII $\longleftrightarrow$ HindIII DNA fragment was made blunt-ended using a DNA polymerase I large fragment according to a customary procedure, and then self-cyclized using T4-DNA ligase as in Example 3. Thus, plasmid pPS1ΔH (about 3.7 Kbp) was constructed wherein a HindIII site present upstream to the promoter region of the penicillinase gene was deleted from plasmid pPS1. Figure 19 shows a process for the construction of the plasmid pPS1ΔH.

Further, plasmid pPS1ΔH was cleaved with a restriction enzyme ClaI according to a customary procedure and mixed with SalI linker having a phosphorylated end (Takara Shuzo). Ligation was carried out using T4-DNA ligase as in Example 3. After ethanol precipitation, the ligation product was cleaved with restriction enzymes HindIII and SalI. After polyacrylamide gel electrophoresis (gel concentration: 5%), a SalI ⟷ HindIII DNA fragment (about 210 bp) containing a promoter region and signal peptide region of the penicillinase gene was recovered by electroelution. On the other hand, plasmid pBR329 was cleaved with restriction enzymes HindIII and SalI in the same manner. After agarose gel electrophoresis (gel concentration: 0.8%), a HindIII ⟷ SalI DNA fragment (about 3.6 Kbp) containing a major portion of vector was recovered. The two DNA fragments thus obtained were mixed, and ligated using T4-DNA ligase as in Example 3, and in the same manner as described above, plasmid p329PS was constructed. Figure 19 shows a process for the construction of the plasmid p329PS.

Example 11 (Construction of plasmid pEXTNF1 which makes possible extracellular secretion of antitumor-active polypeptide)

The human TNF gene expression type plasmid pTNF401NN prepared in Example 4 was cleaved with restriction enzymes AvaI and HindIII in accordance with a customary procedure. After polyacrylamide gel electrophoresis (gel concentration: 5%), an AvaI ⟷ HindIII DNA fragment (about 460 bp) containing a major part of the human TNF gene was recovered. On the other hand, the secretion plasmid vector pEAP8 prepared in Example 9 was cleaved with a restriction enzyme HindIII by a customary procedure. After agarose gel electrophoresis (gel concentration: 0.7%), a linear DNA of pEAP8 was recovered as in Example 3. These two DNA fragments were mixed, termini thereof were made blunted with a DNA polymerase I large fragment by a customary procedure, and then ligation was carried out using a T4-DNA ligase as in Example 3. Escherichia coli HB101 was transformed with the ligated DNA mixture as in Example 3 to obtain a plasmid pEXTNF1 (about 5.2 Kbp)

0317649

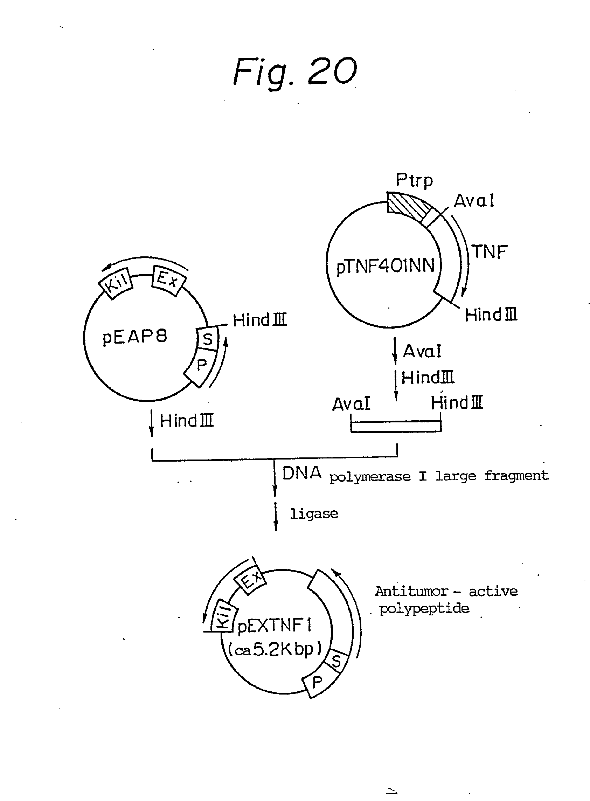

which makes possible an extracellular secretion of the antitumor-active polypeptide gene. This plasmid contains a DNA region coding for a fused protein wherein the antitumor-active polypeptide having the sequence from the 8th Pro to the 157th Leu in the amino acid sequence shown in Figure 1 is linked downstream to the 30th Ala in the amino acid sequence shown in Figure 9. Figure 20 illustrates a process for the construction of pEXTNF1.

Example 12 (Cultivation of recombinant microorganism containing extracellular secretion expression type plasmid pEXTNF1)

The Escherichia coli HB101 obtained in Example 11, i.e., E. coli HB101 containing the plasmid pEXTNF1 which makes possible the extracellular secretion of the antitumor-active polypeptide gene was inoculated in an LBGG medium [1% tryptone, 0.5% yeast extract, 1% NaCl, 0.1% glucose, 0.05 - 0.2% glycerol (pH 7.2)] containing chloramphenical (at a final concentration of 20 µg/ml), and cultivated at 37°C for 24 hours with shaking.

After the cultivation was finishied, the microbial cells were separated by centrifuging, and the resulting culture supernatant was used as a sample for evaluation of activity. Further, all proteins contained in the culture supernatant were precipitated by an ammonium sulfate fractionation (80% saturation). The precipitates obtained by centrifuging were dissolved in an appropriate amount of a PBS buffer (20 mM phosphate buffer containing 150 mM NaCl, pH 7.4), and then dialysis was carried out to a PBS buffer. For the resulting concentrate of the culture supernatant, activity thereof was evaluated.

Example 13 (Activity evaluation of culture supernatant containing antitumor-active polypeptide coded in pEXTNF1)

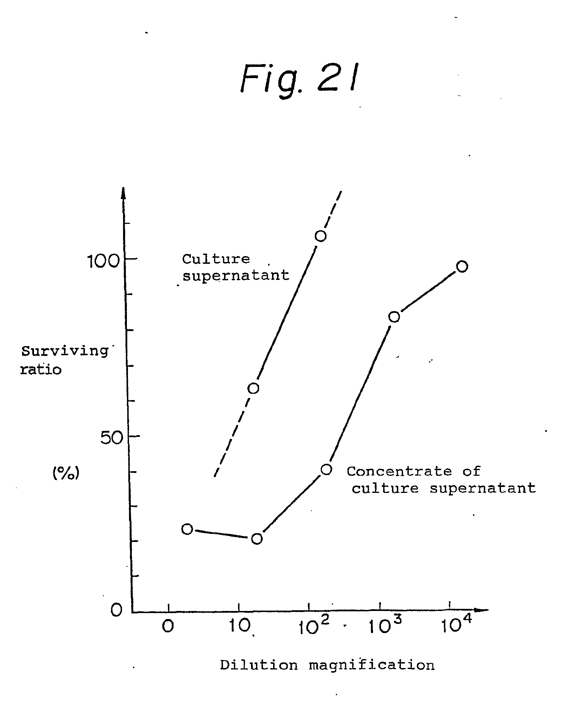

The activity of the culture supernatant containing the antitumor-active polypeptide was measured in accordance with the above-mentioned Ruff method. A series of samples (100 $\mu$l) (diluted with a medium) of the culture supernatant containing the antitumor-active polypeptide or the concentrate thereof, each prepared in Example 12, was mixed with 100 $\mu$l of suspension of mouse L-929 fibroblast (ATCC CCL-929) at a concentration of 4 x $10^5$/ml, in a 96-well microplate (coaster) for tissue culture. To the mixture, actinomycin D (Cosmegen, Banyu Seiyaku) was added at the final concentration of 1 $\mu$g/ml. As a medium, Eagle minimum essential medium (Nissui Seiyaku) containing 5% (vol/vol) of fetal calf serum was used. The cultivation in the above microplate was carried out at 37°C for 20 hours under the air containing 5% carbon dioxide gas, and then, viable cells were stained with a crystal violet solution [solution prepared by dissolving 0.5% (wt/vol) of crystal violet in an aqueous solution of 5% (vol/vol) methanol]. Excess crystal violet was washed out. After drying, remaining crystal violet was extracted with 100 $\mu$l of 0.5% SDS aqueous solution, and an absorbance at 595 nm was measured by ELISA analyzer (Toyo Sokki, ETY-96). The absorbance is proportional to the number of surviving cells. A dilution magnification of the sample exhibiting a 50% level of the absorbance shown by the control not including a diluted sample containing the antitumor-active polypeptide was determined from a graph (Fig. 21), and was designated as a unit. Figure 21 shows that, for the products of Example 12, 100 $\mu$l of the culture supernatant containing the antitumor-active polypeptide exhibits about 10 units of activity, and 100 $\mu$l of the concentrate of the culture supernatant exhibits about 320 units of activity.

An amount of all proteins contained in the culture supernatant (containing the antitumor-active polypeptide) or the concentrate thereof prepared in Example 12 was determined using Protein Assay kit (Bio-

0317649

Rad), and calculated from the standard curve prepared from bovine serum albumin. From the results of the above activity and the protein determination, a specific activity of the concentrate of the culture supernatant containing the antitumor-active polypeptide was calculated to obtain $1.0 \times 10^3$ (unit/mg).

Example 14 (Construction of plasmids pEXTNF3 and pEXTNF4 which can express extracellular secretion of antitumor-active polypeptide gene)

The human TNF gene expression type plasmid TNF401A prepared in Example 4 was cleaved with restriction enzymes AvaI and HindIII in accordance with a customary procedure. After polyacrylamide gel electrophoresis (gel concentration: 5%), an AvaI $\longleftrightarrow$ HindIII DNA fragment (about 460 bp) containing a major part of human TNF gene was recovered.

On the other hand, the secretion plasmid vector pEAP8 prepared in Example 9 was cleaved with the restriction enzyme HindIII according to a customary procedure. After agarose gel electrophoresis (gel concentration: 0.7%), a linear pEAP8 DNA was recovered as in Example 3.

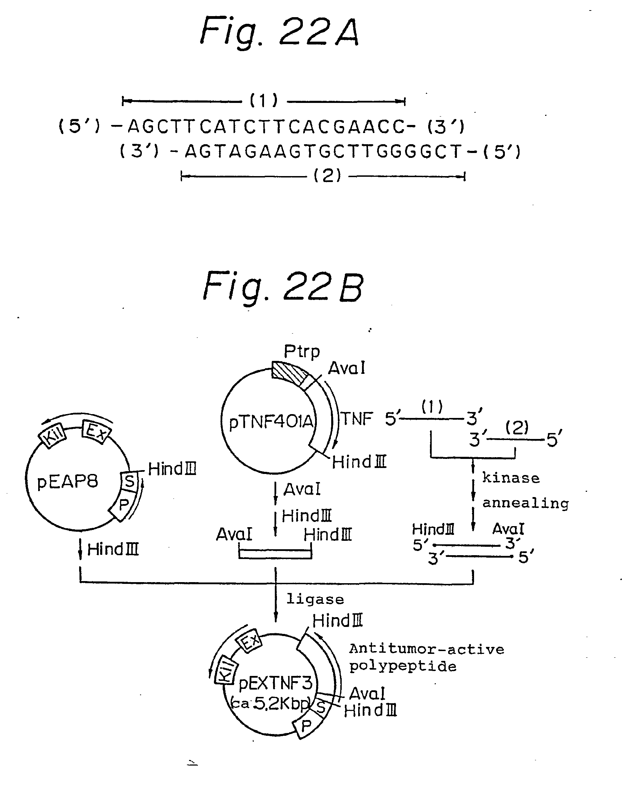

Further, an oligonucleotide having a base sequence shown in Fig. 22A was synthesized and purified as in Example 2. Two synthetic oligonucleotides thus obtained (0.5 μg, each) were phospharylated at the ends thereof as in Example 3, and annealed.

The double-stranded oligonucleotide obtained by the above annealing was mixed with the previously obtained linear pEAP8 DNA fragment (HindIII $\longleftrightarrow$ HindIII) and the DNA fragment (AvaI $\longleftrightarrow$ HindIII) (about 460 bp) containing a major part of human TNF gene. After ethanol precipitation, ligation was carried out with T4-DNA ligase as in Example 3. Escherichia coli HB101 was transformed with the ligated DNA mixture as in Example 3 to obtain plasmid pEXTNF3 (about 5.2 Kbp) which can express the extracellular secretion of the antitumor-active polypeptide gene. This plasmid contains a DNA region coding for a fused protein wherein the antitumor-active polypeptide having a sequence from the third Ser to the 157th Leu in the amino acid sequence shown in Figure 1 is linked downstream to the 30th Ala in the amino acid sequence shown in Figure 9. Figure 22 illustrates a process for the construction of pEXTNF3.

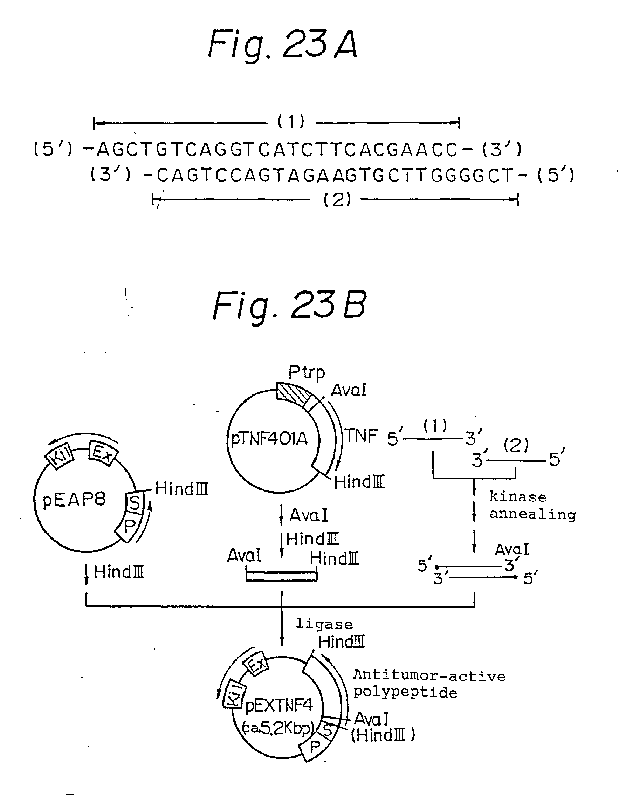

Further, according to the same procedure as above, the oligonucleotide shown in Figure 23 was used to construct plasmid pEXTNF4 (about 5.2 Kbp) which can express the extracellular secretion of the antitumor-active polypeptide gene. This plasmid contains a DNA region coding for a fused protein wherein the antitumor-active polypeptide having a sequence from the first Val to the 157th Leu in the amino acid sequence shown in Figure 1 is linked downstream to the 30th Ala of the amino acid sequence shown in Figure 9. Figure 23 shows a process for the construction of pEXTNF4.

Example 15    (Cultivation of recombinant microorganism containing various plasmids
              which makes possible extracellular
              secretion)

Escherichia coli HB101 containing the secretion plasmid vector pEAP8 (prepared in Example 9), Escherichia coli HB101 containing the plasmid pEXTNF1 which makes possible the extracellular secretion of the antitumor-active polypeptide gene (prepared in Example 11), or Escherichia coli HB101 containing the plasmid pEXTNF3 or pEXTNF4 which makes possible the extracellular secretion of the antitumor-active polypeptide (prepared in Example 14) was inoculated in an LBG medium [1% tryptone, 0.5% yeast extract, 1% NaCl, 0.1% glucose (pH 7.2)], and cultivated at 37°C for 24 hours with shaking, respectively.

After the cultivation was finished, cells were separated by centrifuging, and the resulting culture supernatants were used as samples for evaluating activity.

Example 16   (Evaluation of carcinostatic activity in vitro of culture supernatant containing various antitumor-active polypeptides)

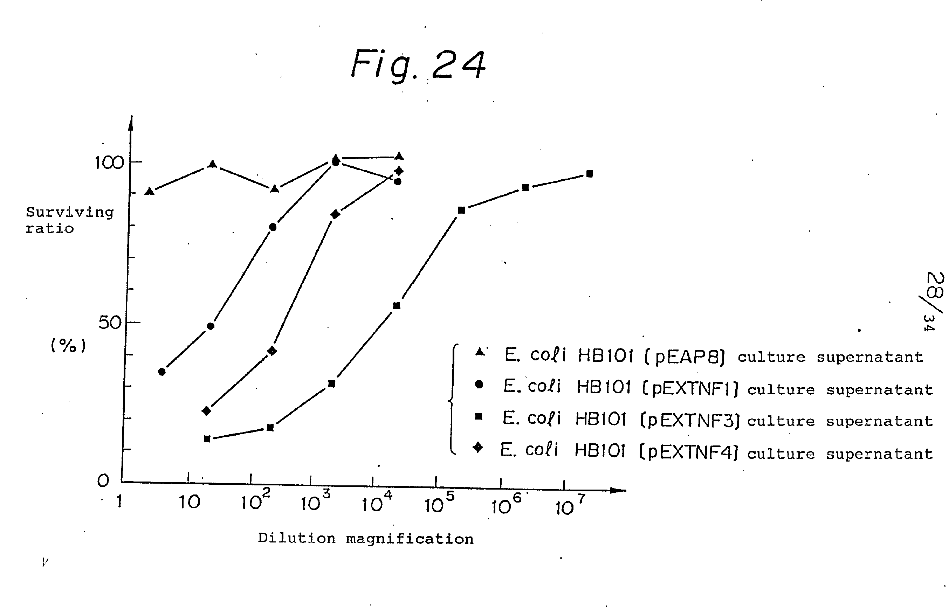

For the culture supernatants containing various antitumor-active polypeptides (obtained in Example 13), carcinostatic activity in vitro was measured as in Example 13.   Figure 24 shows that 100 μl of the culture supernatant from Escherichia coli containing plasmid pEXTNF1 exhibits about 38 units of activity, 100 μl of the culture supernatant from Escherichia coli containing plasmid pEXTNF3 exhibits about 11,000 units of activity, and 100 μl of the culture supernatant from Escherichia coli containing plasmid pEXTNF4 exhibits about 330 units of activity.   The culture supernatant from Escherichia coli containing secretion plasmid vector pEAP8 does not exhibit any activity.

An amount of all proteins contained in the culture supernatants containing various antitumor-active polypeptides (prepared in Example 15) was determined using Protein Assay kit (Bio-Rad), and calculated from a standard curve prepared from bovine serum albumin.   From the results of the above activities and the protein determination, a specific activity of each of the culture supernatants containing the antitumor-active polypeptide was calculated to obtain the results listed in Table 1.

Table 1

| Plasmid | pEAP8 | pEXTNF1 | pEXTNF3 | pEXTNF4 |
|---|---|---|---|---|
| Activity (U/ml) | 0 | $3.8 \times 10^2$ | $1.1 \times 10^5$ | $3.3 \times 10^3$ |
| Protein (mg/ml) | 0.65 | 0.63 | 0.32 | 0.52 |
| Specific activity (U/mg) | 0 | $6.0 \times 10^2$ | $3.4 \times 10^5$ | $6.3 \times 10^3$ |

To concentrates prepared from 100 $\mu$l of the culture supernatants of Escherichia coli HB101 containing pEAP8 and Escherichia coli HB101 containing pEXTNF3, were added Tris-HCl buffer (pH 6.8), SDS, 2-mercaptoethanol and glycerol so that the final concentrations thereof were 60 mM, 2%, 4% and 10%, respectively. SDS-polyacrylamide gel electrophoresis was carried out [Suzuki, Iden (Inheritance), 31, 43 (1977)]. The concentration of a separating gel was 15%, and SDS-Tris-glycine was used as a migration buffer [U.K. Laemmli, Nature, 227, 680 (1970)]. After electrolysis was completed, proteins in the gel were transferred to a nitrocellulose filter, in an electrophoresis manner, in a buffer of 25 mM Tris-192 mM glycine (pH 8.3)-20% methanol, to perform western blotting.

The nitrocellulose filter to which proteins were transferred was dipped in a PBS buffer containing 5% bovine serum abbumin for 60 minutes, and then, the antitumor-active polypeptide in the culture supernatant was specifically strained, by means of Immunblot assay kit (Bio-Rad) using an antibody labeled with peroxidase, in accordance with an indirect method using as a primary antibody a goat antihuman TNF protein antiserum [prepared by immunizing a goat with human TNF produced in cells of Escherichia coli (prepared in Example 17 below) as an antigen (Otsuka Assei Kenkyujo). As a control, a lysate of Escherichia coli C600r$^-$m$^-$

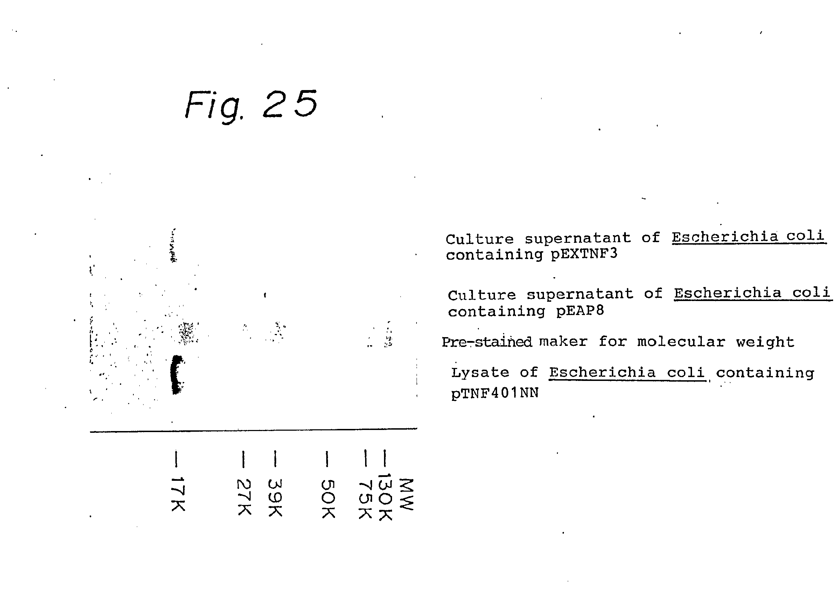

containing pTNF401NN was used. Figure 25 shows a copy of a part of the results. A band caused from the antitumor-active polypeptide was detected in the culture supernatant of Escherichia coli containing pEXTNF3.

It is apparent from the results shown in Table 1 and figure 25 that the extracellular secretion of the antitumor-active polypeptide can be very efficiently performed by fusing the antitumor-active gene having serine at the amino terminus thereof, downstream to the signal region of penicillinase gene of alkalophilic cell.

Example 17   (Purification of antitumor-active polypeptide)

Escherichia coli C600r⁻m⁻ containing human TNF gene expression type plasmid pTNF401NN (prepared in Example 4) was inoculated in 200 ml of an M9 medium [prepared by adding $MgSO_4$ aqueous solution and $CaCl_2$ aqueous solution sterilized in an autoclave at the final amounts of 2 mM and 0.1 mM, respectively to an aqueous solution sterilized in an autoclave and containing 0.6% $Na_2HPO_4$-0.3%$KH_2PO_4$-0.05%NaCl-0.1%$NH_4$Cl (pH 7.4)] containing 30 μg/ml of ampicillin, 0.2% glucose and 4 mg/ml at casamino acid. Cultivation was continued at 37°C with shaking, until $OD_{600}$ reached to 0.7. Then, 50 μg/ml (final concentration) of 3-β-indoleacrylic acid was added to the medium, and the cultivation was continued at 37°C for further 12 hours.

Cells of Escherichia coli were harvested by centrifuging, and then washed with a PBS buffer (20 mM phosphate buffer containing 150 mM NaCl, pH 7.4). The washed cells were suspended in 10 ml of a PBS buffer, and disrupted, using a sonicator (Kubota, 200 M). Then, the cell debris were removed by centrifugation.

Human TNF protein was purified from the resulting lysate, using DEAE-Sepharose column chromatography in accordance with a method of Shirai, et al [T. Shirai, et al., Nature, 313, 830 (1985)]. The content of human TNF

0317649

protein in the resulting crude product was about 30%.

A mouse hydridoma to produce a monoclonal antibody to human TNF protein was obtained in accordance with a method of Kobler and Milstein [Kobler and Milstein, Nature, 256, 495 (1975)]. Male Balb/c mouse was immunized with the above crude human TNF product. The spleen cell from the mouse and a mouse myeloma cell P3-X63-Ag8-UI [O.E. Yelton, et al., Current Topics in Microbiology and Immunology, 81, 1 (1978)] were fused. A mixture of the fused cells was cultivated in a screening medium to select only hydridoma cell. For the antibody in the culture supernatant, a binding ability to the crude human TNF product was determined, and the clone producing the antibody to human TNF protein was obtained.

The monoclonal antibody was purified from the culture supernatant of the mouse hydridoma producing the monoclonal antibody to human TNF protein, using Protein A Sepharose column chromatography (Pharmacia). The resulting purified monoclonal antibody was coupled with active affinity support Affi-Gel 10 (Bio-Rad) at 4°C for 2 hours in 0.1M MOPS buffer (pH 7.5, Nakarai Kagaku Yakuhin) to obtain an affinity column for purifying human TNF protein and antitumor-active polypeptide.

The culture supernatant of Escherichia coli (prepared in Example 15) containing the plasmid pEXTNF3 which makes possible the extracellular secretion of the antitumor-active polypeptide gene was passed through the affinity column, whereby only the antitumor-active polypeptide was specifically adsorbed to the column. The column was thoroughly washed with a PBS buffer and 20 mM phosphate buffer (pH 7.4) containing 500 mM NaCl, and then the antitumor-active polypeptide was eluted, using 3M NaSCN aqueous solution (pH 7.4). The eluted polypeptide having antitumor-activity was concentrated by ultrafiltration, poured into a PBS buffer and then

- 43 -

subjected to SDS-polyacrylamide gel electrophoresis (separating gel concentration: 15%). After electrophoresis, protein in the gel was stained. Only one band was observed at a molecular weight of about 17,000. It was confirmed therefrom that the antitumor-active polypeptide secreted outside the cells of <u>Escherichia coli</u> was obtained in a purity above 98%.

According to the same procedure as above, lysate of <u>Escherichia coli</u> containing TNF401NN (prepared as above) was treated with the affinity column to purify human TNF protein and obtain the purified human TNF protein.

Example 18 (Properties of antitumor-active polypeptide secreted)

The purified antitumor-active polypeptide secreted outside the cells of <u>Escherichia coli</u> (prepared in Example 17) was tested with a vapor phase protein sequencer (ABI, Model 470A) to analyze an amino acid sequence at the amino terminus. The results was as follows:

$H_2$N-Ser-Ser-Ser-Arg-Thr-Pro-Ser-Asp- ... ...

The above result shows that the signal peptide of penicillinase was accurately cleaved during secretion. Further, an analysis result of composition of the amino acids supports that the antitumor-active polypeptide secreted outside the cells of <u>Escherichia coli</u> corresponded to one designed.

The purified human TNF protein was analyzed on an amino acid sequence at the amino terminus. As the amino acid at the amino terminus, methionine, valine, arginine and serine were detected. This means that human TNF protein produced in <u>E. coli</u> cells has various amino termini.

The carcinostatic activity in vitro of each of the purified products was determined as in Example 13. Human TNF protein produced in <u>E. coli</u> cells had a specific activity of $1 \times 10^7$ (unit/mg), and the antitumor-active polypeptide secreted outside the cells had a specific activity of 2 to 4 x $10^7$ (unit/mg).

It is apparent from the above results that the antitumor-active polypeptide which is homogeneous and has high specific activity can be obtained by the extracellular secretion.

Example 19 (Evaluation of carcinostatic activity in vivo of antitumor-active polypeptide secreted)

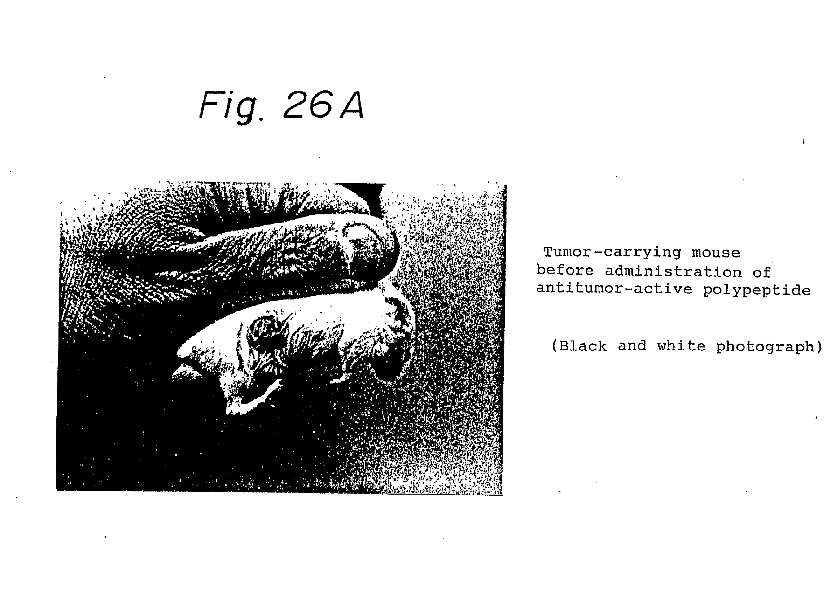

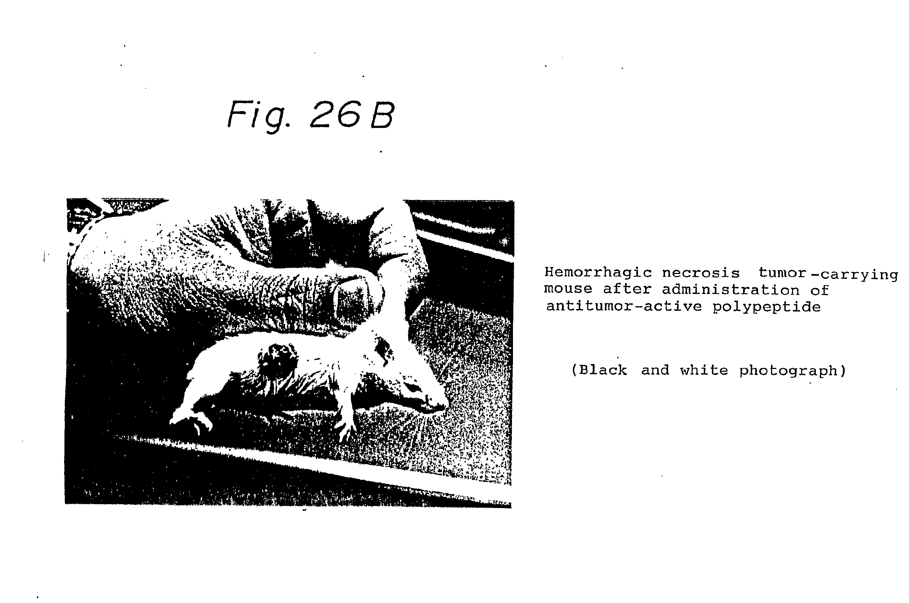

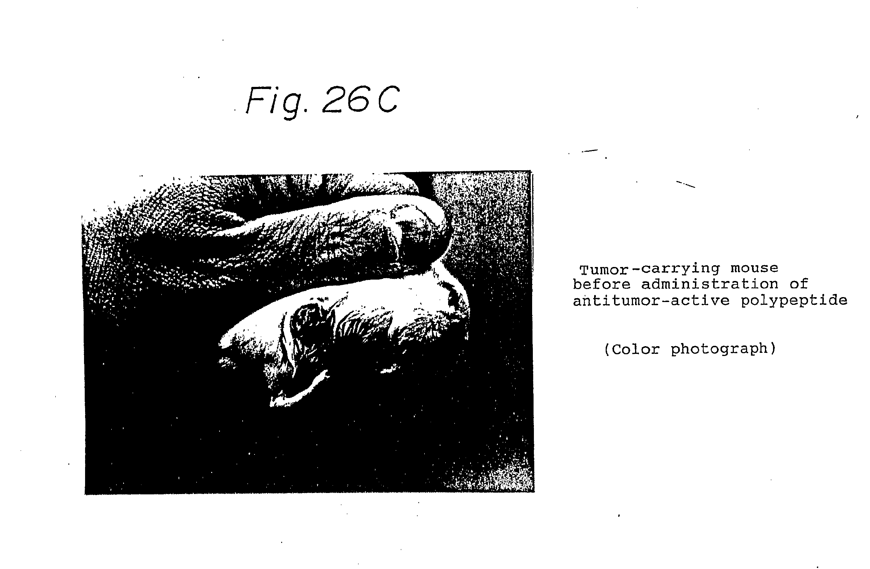

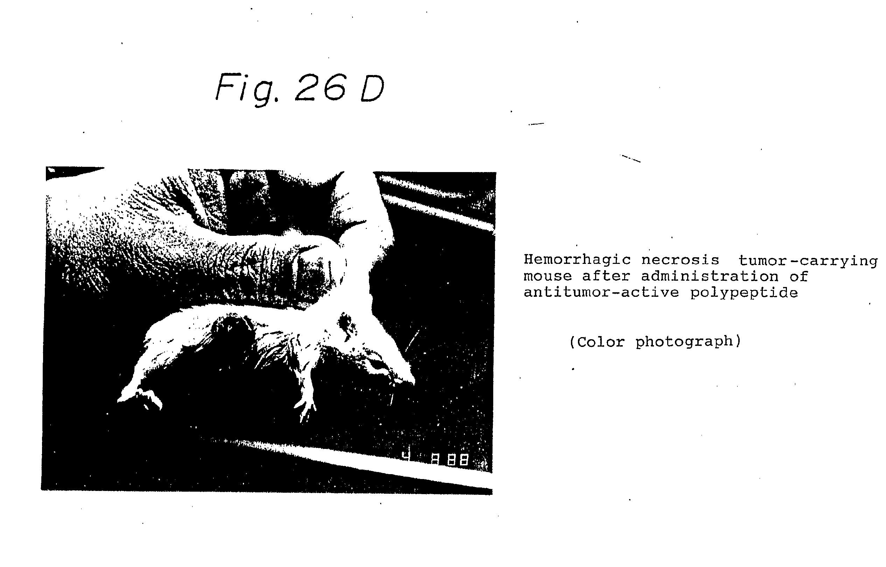

For the antitumor-active polypeptide secreted, carcinostatic activity in vivo was measured in accordance with a process of Carswell, et al., Meth A sarcoma cells (1 x $10^6$) were suspended in 50 $\mu$l of an RPMI 1640 medium (Nissui), and then transparent to BALB/c mouse (6 - 8 weeks, male, Charles River) at a subcutaneous part of latus. When the diameter of the tumor reached to 6 - 10 mm 7 - 10 days after transplantation, 10 $\mu$g of the antitumor-active polypeptide secreted was administrated through a vein in a tail. In 24 hours after administration, hemorrhagic necrosis was observed on the surface of the tumor. This means that the secreted antitumor-active polypeptide according to the present invention has a remarkable carcinostatic activity in vivo. Figure 26 shows an example of each of a tumor-carrying mouse before administration, and a mouse having a hemorrhagic necrosis tumor. Further, 10 $\mu$g of the antitumor-active polypeptide secreted, human TNF protein produced in the cells, or physiological saline was administrated in the tumor carrying mouse through a vein of a tail. According to the above-mentioned method of Carswell, et al., the degree of hemorrhagic necrosis 24 hours after the administration was compared. The result is shown in Table 2. It is apparent from Table 2 that the activity in vivo of the antitumor-active polypeptide secreted is higher than that of the human TNF protein produced in E. coli cells, as the activity in vitro.

0317649

Table 2

| | Degree of hemorrhagic necrosis (Numbers of mouse) | | | |
|---|---|---|---|---|
| | − | + | ++ | +++ |
| Control (physiological saline) | 6 | 0 | 0 | 0 |
| Human TNF protein produced in E. coli cells | 0 | 5 | 2 | 0 |
| Antitumor-active polypeptide secreted | 0 | 1 | 3 | 4 |

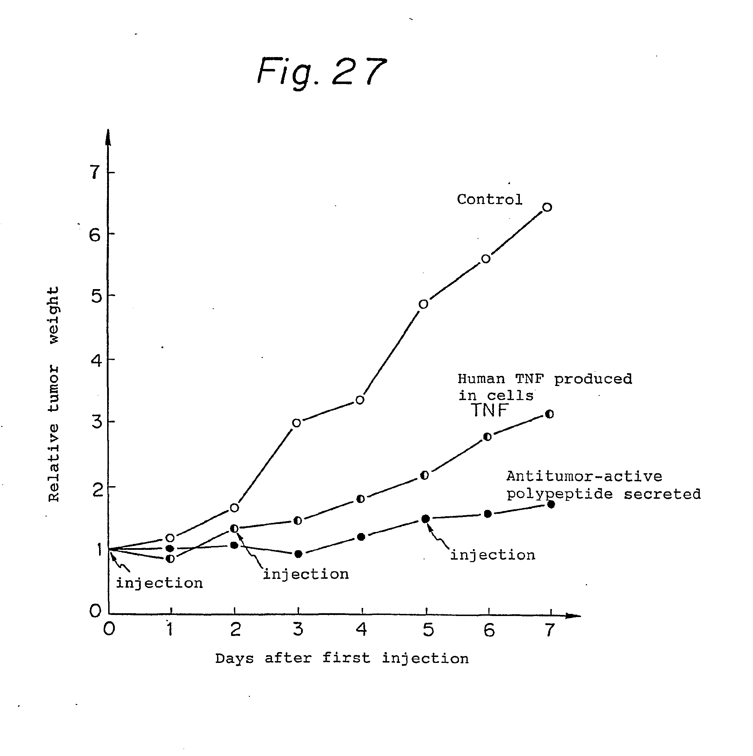

Further, 10 µg of the antitumor-active polypeptide secreted or human TNF protein produced in cells was administrated twice to the tumor-carrying mouse as above through a vein of a tail. A change of relative weight of the tumor was measured. Figure 27 shows the results. In Figure 27, the tumor weight is calculated from $1/2$ x (major axis of tumor) x (minor axis of tumor)$^2$. It is apparent from the above results that the antitumor-active polypeptide secreted is more effective than human TNF protein produced in E. coli cells. A factor of a thickness is neglected in the calculation equation for the relative tumor weight. However, when the antitumor-active polypeptide secreted was administrated, the thickness of the tumor was reduced remarkably, in comparison with the administration of human TNF protein produced in E. coli cells. Therefore, the difference in activity between the antitumor-active polypeptide secreted and human TNF protein produced in E. coli cells is in fact larger than that shown in Figure 27.

Although the present invention has been described with reference to specific embodiments, various modification obvious to those skilled in the art is within the

0317649

scope of the present invention.

[Reference to Deposited Microorganism according to Rule 13 bis]

i) Depository Authority

Name: Fermentation Research Institute, Agency of Industrial Science and Technology, Ministry of International Trade and Industry

Address: 1-3 Higashi 1 chome, Tsukuba-shi, Ibaraki-ken, Japan (Mail number 305)

ii) Deposition Date
June 10, 1988

iii) Deposition Number
Bikoken Joki 1909
(FERM BP-1909)

## CLAIMS

1. A plasmid comprising

(1) a first DNA region comprising a DNA sequence coding for a polypeptide having an antitumor activity, a DNA sequence having a promoter function for controlling an expression of said polypeptide, and a DNA sequence coding for a signal peptide, and

(2) a second DNA region comprising a DNA sequence causing host cells to facilitate extracellular secretion, and a DNA sequence having a promoter function for controlling an expression of said DNA sequence.

2. A plasmid according to claim 1, wherein the DNA sequence coding for the polypeptide having an antitumor activity contains at least a DNA sequence coding for a polypeptide containing the amino acid sequence shown in Fig. 1, or a DNA sequence coding for a polypeptide containing the amino acid sequence wherein one or more amino acid groups are replaced in, deleted from or inserted to the amino acid sequence shown in Fig. 1.

3. A plasmid according to claim 1, wherein said DNA sequence having the promoter function in the first DNA region is derived from a chromosomal DNA of an alkalophilic Bacillus strain No. 170.

4. A plasmid according to claim 1, wherein the DNA sequence coding for a signal peptide is derived from the chromosomal DNA of alkalophilic Bacillus strain No. 170.

5. A plasmid according to claim 1, wherein the DNA sequence causing host cells substantially to facilitate the extracellular secretion is derived from plasmid pMB9.

6. A plasmid according to claim 1, wherein the DNA sequence having the promoter function in the second DNA region is derived from chromosomal DNA of alkalophilic Bacillus strain No. 170.

7. A plasmid according to claim 1, which is a

plasmid pEXTNF1, pEXTNF3 or pEXTNF4.

8. A recombinant microbial cell transformed with a plasmid comprising

(1) a first DNA region comprising a DNA sequence coding for a polypeptide having an antitumor activity, a DNA sequence having a promoter function for controlling an expression of said polypeptide, and a DNA sequence coding for a signal peptide, and

(2) a second DNA region comprising a DNA sequence causing host cells to facilitate extracellular secretion, and a DNA sequence having a promoter function for controlling an expression of said DNA sequence.

9. A microbial cell according to claim 8, wherein the microbial cell is of the genus Escherichia.

10. A microbial cell according to claim 8, wherein the microbial cell is of Escherichia coli HB101.

11. A process for production of a polypeptide having an antitumor activity, characterized in that a recombinant microbial cell transformed with a plasmid comprising

(1) a first DNA region comprising a DNA sequence coding for a polypeptide having an antitumor activity, a DNA sequence having a promoter function for controlling an expression of said polypeptide, and a DNA sequence coding for a signal peptide, and

(2) a second DNA region comprising a DNA sequence causing host cells to facilitate extracellular secretion, and a DNA sequence having a promoter function for controlling an expression of said DNA sequence is cultivated until the polypeptide having an antitumor activity is formed and accumulates outside the cells, and recovering the polypeptide having an antitumor activity from the cultured broth.

12. A process according to claim 11, wherein the microbial cell is of the genus Escherichia.

13. A process according to claim 11, wherein the microbial cell is of Escherichia coli HB101.

14. A polypeptide having an antitumor activity, which is secreted from a recombinant microbial cell transformed with a plasmid comprising

(1) a first DNA region comprising a DNA sequence coding for a polypeptide having an antitumor activity, a DNA sequence having a promoter function for controlling an expression of said polypeptide, and a DNA sequence coding for a signal peptide, and

(2) a second DNA region comprising a DNA sequence causing host cells to facilitate extracellular secretion, and a DNA sequence having a promoter function for controlling an expression of said DNA sequence.

15. A polypeptide according to claim 14, containing the amino acid sequence shown in Fig. 1.

16. A polypeptide according to claim 14, containing a DNA sequence coding for a polypeptide containing the amino acid sequence wherein one or more amino acids are replaced by, deleted from or inserted to the amino acid sequence shown in Fig. 1.

17. A polypeptide according to claim 14, secreted from a microbial cell belonging to the genus _Escherichia_ and transformed with a plasmid pEXTNF1, pEXTNF3 or pEXTNF4.

# Fig. IA

```
ClaI
|
TCG-ATA-ATG-GTC-AGG-TCA-TCT-TCA-CGA-ACC-CCG-AGT-GAC-AAG-CCT-GTA-GCC-CAT-GTT-GTA
        (MET)Val-Arg-Ser-Ser-Ser-Arg-Thr-Pro-Ser-Asp-Lys-Pro-Val-Ala-His-Val-Val
            1                                              10


GCA-AAC-CCT-CAA-GCT-GAG-GGG-CAG-CTC-CAG-TGG-CTG-AAC-CGC-CGG-GCC-AAT-GCC-CTG-CTG
Ala-Asn-Pro-Gln-Ala-Glu-Gly-Gln-Leu-Gln-Trp-Leu-Asn-Arg-Arg-Ala-Asn-Ala-Leu-Leu
            20                                              30


GCC-ATT-GGC-GTG-GAG-CTG-AGA-GAT-AAC-CAG-CTG-GTG-GTA-CCA-TCA-GAG-GGC-TTG-TAC-CTC
Ala-Asn-Gly-Val-Glu-Leu-Arg-Asp-Asn-Gln-Leu-Val-Val-Pro-Ser-Glu-Gly-Leu-Tyr-Leu
            40                                              50


ATT-TAC-TCC-CAG-GTC-CTC-TTC-AAG-GGC-CAA-GGC-TGC-CCG-TCG-ACC-CAT-GTG-CTC-CTC-ACC
Ile-Tyr-Ser-Gln-Val-Leu-Phe-Lys-Gly-Gln-Gly-Cys-Pro-Ser-Thr-His-Val-Leu-Leu-Thr
            60                                              70


CAC-ACC-ATC-AGC-CGC-ATC-GCC-GTC-TCC-TAC-CAG-ACC-AAG-GTC-AAC-CTC-CTC-TCT-GCG-ATC
His-Thr-Ile-Ser-Arg-Ile-Ala-Val-Ser-Tyr-Gln-Thr-Lys-Val-Asn-Leu-Leu-Ser-Ala-Ile
            80                                              90


AAG-AGC-CCC-TGC-CAG-AGG-GAG-ACC-CCA-GAG-GGG-GCT-GAG-GCC-AAG-CCA-TGG-TAT-GAG-CCC
Lys-Ser-Pro-Cys-Gln-Arg-Glu-Thr-Pro-Glu-Gly-Ala-Glu-Ala-Lys-Pro-Trp-Tyr-Glu-Pro
            100                                             110


ATC-TAT-CTG-GGA-GGG-GTC-TTC-CAG-CTG-GAG-AAG-GGT-GAC-CGA-CTC-AGC-GCT-GAA-ATC-AAT
Ile-Tyr-Leu-Gly-Gly-Val-Phe-Gln-Leu-Glu-Lys-Gly-Asp-Arg-Leu-Ser-Ala-Glu-Ile-Asn
            120                                             130
```

```
CGG-CCC-GAC-TAT-CTC-GAC-TTT-GCC-GAG-TCT-GGG-CAG-GTC-TAC-TTT-GGG-ATT-ATT-GCC-CTG
Arg-Pro-Asp-Tyr-Leu-Asp-Phe-Ala-Glu-Ser-Gly-Gln-Val-Tyr-Phe-Gly-Ile-Ile-Ala-Leu
        140                                           150

        HindIII
          |
TGA-TAA-GCT
***-***
```

## Fig. 2

```
                                        ┤──────────── TNF-1 ──────────────────────────────────────────────────────────┤
CGATAATGGTCAGGTCATCTTCACGAACCCCGAGTGACAAGCCTGTAGCCCATGTTGTAGCAAACCCTCAAGCTGAGGGGC
 TATTACCAGTCCAGTAGAAGTGCTTGGGGCTCACTGTTCGGACATCGGGTACAACATCGTTTGGGAGTTCGACTCCCCG
├────────────────────────────────── TNF-2 ──────────────────────────────────────┤

┤─────────────────────── TNF-3 ──────────────┤
AGCTCCAGTGGCTGAACCGCCGGGCCAATGCCCTGCTGGCCAATGGCGTGGAGCTGAGAGATAACCAGCTGGTGGTACCAT
TCGAGGTCACCGACTTGGCGGCCCGGTTACGGGACGACCGGTTACCGCACCTCGACTCTCTATTGGTCGACCACCATGGTA
├───────── TNF-4 ─────────┤                            ├────────TNF-5────────────┤

┤──────── TNF-6 ────────────────────────────────┤
CAGAGGGCTTGTACCTCATTTACTCCCAGGTCCTCTTCAAGGGCCAAGGCTGCCCGTCGACCCATGTGCTCCTCACCCACA
GTCTCCCGAACATGGAGTAAATGAGGGTCCAGGAGAAGTTCCCGGTTCCGACGGGCAGCTGGGTACACGAGGAGTGGGTGT
├──┤                    ├──────────────── TNF-7 ──────────────────┤

┤─────────────── TNF-8 ──────────────┤                        ├────────── TNF-10 ─────────┤
CCATCAGCCGCATCGCCGTCTCCTACCAGACCAAGGTCAACCTCCTCTCTGCGATCAAGAGCCCCTGCCAGAGGGAGACCC
GGTAGTCGGCGTAGCGGCAGAGGATGGTCTGGTTCCAGTTGGAGGAGAGACGCTAGTTCTCGGGGACGGTCTCCCTCTGGG
├─────────────── TNF-9 ──────────────┤                        ├────────── TNF-11 ─────────┤

┤──┤                    ├───────────── TNF-12 ──────────────┤                    ├──── TNF-14 ──┤
CAGAGGGGGCTGAGGCCAAGCCATGGTATGAGCCCATCTATCTGGGAGGGGTCTTCCAGCTGGAGAAGGGTGACCGACTCA
GTCTCCCCCGACTCCGGTTCGGTACCATACTCGGGTAGATAGACCCTCCCCAGAAGGTCGACCTCTTCCCACTGGCTGAGT
├──┤                  ├──────────────── TNF-13 ──────────────┤

┤──────────┤                           ├──────────────────── TNF-16 ────────────────────────┤
GCGCTGAAATCAATCGGCCCGACTATCTCGACTTTGCCGAGTCTGGGCAGGTCTACTTTGGGATTATTGCCCTGTGATA
CGCGACTTTAGTTAGCCGGGCTGATAGAGCTGAAACGGCTCAGACCCGTCCAGATGAAACCCTAATAACGGGACACTATTCGA
├── TNF-15 ──────────────┤              ├──────────────────── TNF-17 ────────────────────────┤
```

Fig. 3

# Fig. 4

# Fig. 5

Fig. 6

# Fig. 7A

```
                    |————————————— (1) —————————————|
(5')- A G C T T A G C C C G C C T A A T G A G C G G G C T T T T T T T T -(3')
   (3')- A T C G G G C G G A T T A C T C G C C C G A A A A A A A A A -(5')
        |————————————————— (2) —————————————|
```

# Fig. 7B

Fig. 8

## Fig. 9

TTTAAAGCGTAGAAAATTTTGTACGCTTTTTTGTTAATTACATAAAAGTATGCAAATGAAGATGGAACAACATTTGA

GATGAATTGTCTAATATAGGTAATAACTATTTAGCTTGAAAGAAAGGGTTGATAACATG-AAA-AAG-AAT-ACG-TTG-
                                                          MET-Lys-Lys-Asn-Thr-Leu-
                                                              (1)

                                           50
TTA-AAA-GTA-GGA-TTA-TGT-GTA-AGT-TTA-CTA-GGA-ACA-ACT-CAA-TTT-GTT-AGC-ACG-ATT-TCT-
Leu-Lys-Val-Gly-Leu-Cys-Val-Ser-Leu-Leu-Gly-Thr-Thr-Gln-Phe-Val-Ser-Thr-Ile-Ser-
            (10)                                              (20)

        HindIII
TCT-GTA-CAA-GCT-T---
Ser-Val-Gln-Ala----
            (30)

signal peptide

## Fig. 10

Kil  gene

ATGAGGAAAAGATTTTTTGTGGGAATATTCGCGATAAACCTCCTTGTTGGATGTCAGGCTAACTATATACGTGATGTTCAG
MetArgLysArgPhePheValGlyIlePheAlaIleAsnLeuLeuValGlyCysGlnAlaAsnTyrIleArgAspValGln


    90                                                       138
GGAGGGACCATCGCACCATCCTCCTCTTCTAAACTGACGGGGATCGCGGTTCAGTAG
GlyGlyThrIleAlaProSerSerSerSerLysLeuThrGlyIleAlaValGln***

# Fig. 11

Ex promoter

HincII                                                  -35EcoRV        60
GTCAACAATA TGAACTGTCA CAAATCTTAT ATATATATTG TGA TTGATAT CACATCACTT

          -10              ┌─mRNA                                      120
TTTTTCAATG GG TATTAT GC TTAA GGTGTA ATGAATGATT GGGAGAGGGT GGGATGATAT

                                                                       180
GTTTTGTTAT CAATGTGAAC AAAGCCAACA GGCGGTTGTA AAGTAATGGG TGTTTGTGGC

                                                                       240
GAAGAATGAA ACGATTGCGA GTTTACAAGA TACGATTGTG TTTGGGCTAA AAGGAATTGC

                                                                       300
AGCTTATCGC ACACATGCTG CTCAGCTAGG GTATACGGAT GCATTTGTAG ATGCTACAAC

HindIII    311
ACAAGAAGCT T ──────Kil gene ──────

# Fig. 12 (1)

CELLS
  | SUSPENDING IN 10 ml OF SOLUTION CONTAINING 20 % SUCROSE,
  | 50 mM Tris-HCl (pH 8.0) AND 1 mM EDTA ON ICE BATH
  ↓

50 ml POLYPROPYLENE CENTRIFUGE TUBE

  | 2 ml OF 0.25 M EDTA
  | 1 ml OF LYSOZYME [5 mg/ml, 0.025 M Tris-HCℓ (pH 8.0)]

  | 0.1 ml OF  RIBONUCLEASE A (10 mg/ml)
  ↓

LYSIS   MIXING GENTLY AND STANDING FOR 15 TO 30 MIN. IN ICE
  |     BATH.

  | 5 ml 3X Triton X-100 AND GENTLY MIXING AND STANDING FOR
  | 15 TO 45 MIN. ON ICE BATH.

  ↓

CENTRIFUGATION AT 17,000 r.p.m., 4°C, FOR 40 MIN.
  ↓
SUPERNATANT
  ↓
250 ml GLASS BOTTLE

  | 2/3 VOLUME OF DISTILLED WATER (TWICE DISTILLED)

  | 2/3 VOLUME OF COLD WATER-SATURATED PHENOL AND MIXING GENTLY

  ↓

CENTRIFUGATION AT 6,500 r.p.m., 4°C, FOR 15 MIN.
  ↓

# Fig. 12 (2)

SUPERNATANT

   EQUAL VOLUME OF PHENOL/CHLOROFORM

CENTRIFUGATION AT 6,500 r.p.m., 4°C, FOR 15 MIN.

SUPERNATANT

   1/25 VOLUME OF 5 M NaCl

   TWICE VOLUME OF ETHANOL AND STANDING AT -20°C OVERNIGHT

CENTRIFUGATION AT 6,500 r.p.m., -20°C, FOR 60 MIN.

DNA PELLET ELIMINATING EXCESS LIQUID

   REDISSOLVING IN 5 ml OF A-50 BUFFER

   1 ml OF STERILIZED 80% GLYCEROL AND GENTLY MIXING

A-50 COLUMN (2 x 35 cm, 1 FRACTION = 4 ml)

DNA FRACTION ($A_{260}$ PEAK)

   TWICE VOLUME ETHANOL AND STANDING AT -20°C OVERNIGHT

# Fig. 12 (3)

CENTRIFUGATION AT 6,500 r.p.m., -20°C, FOR 60 MIN.

DNA PELLET

   2.1 ml OF TEN BUFFER [20 mM Tris-HCl (pH 7.5) 50 mM NaCl, 1 mM EDTA] (IN 5 ml NITROCELLULOSE TUBE) 2.2 g OF CsCl AND MIXING

   (KEEPING IN DARK)

   150 µl OF PbI (2 mg/ml) AND THOROUGHLY MIXING 2 ml OF MINERAL OIL

CsCl GRADIENT CENTRIFUGATION AT 36,000 r.p.m., 20°C FOR 40 HRS.

   OBSERVATION BY UV
      UPPER BAND:  CHROMOSOMAL NICKED DNA
      LOWER BAND:  COVALENTLY CLOSED PLASMID DNA

COLLECTING DROPWISE LOWER BAND DNA

DOWEX 50W-X8 COLUMN (DETECTION BY UV)

   (KEEPING IN LIGHT)

# Fig. 12 (4)

DIALYSIS IN 2 TO 4 ℓ OF BUFFER CONTAINING 10 mM Tris-HCl

(pH 8.0) AND 1 mM EDTA AT 4°C OVERNIGHT

30 ml CORTEX CENTRIFUGE TUBE

1/25 VOLUME OF 5 M NaCl

TWICE VOLUME OF ETHANOL AND STANDING AT -20°C OVERNIGHT

CENTRIFUGATION AT 6,500 r.p.m., -20°C, FOR 60 MIN.

DNA PRECIPITATION

1 TO 2 ml OF TEN BUFFER

PURIFIED PLASMID DNA (1 mg/1 ml CULTURE) STORAGE AT -20 TO -70°C

# Fig. 13

# Fig.14

# Fig. 15

# Fig. 16

0317649

# Fig. 17

# Fig. 18

# Fig. 19

# Fig. 20

# Fig. 2I

Surviving ratio

(%)

Culture supernatant

Concentrate of culture supernatant

Dilution magnification

# Fig. 22A

```
        |————————— (1) —————————|
(5') -AGCTTCATCTTCACGAACC- (3')
     (3') -AGTAGAAGTGCTTGGGGCT-(5')
        |————————— (2) —————————|
```

# Fig. 22B

# Fig. 23A

```
          |←————————— (1) —————————→|
(5') -AGCTGTCAGGTCATCTTCACGAACC- (3')
        (3') -CAGTCCAGTAGAAGTGCTTGGGGCT- (5')
         |←——————————— (2) ———————————→|
```

# Fig. 23B

# Fig. 24

Surviving
ratio

(%)

100

50

0

Dilution magnification

1  10  10² 10³ 10⁴ 10⁵ 10⁶ 10⁷

▲ E. coli HB101 [pEAP8] culture supernatant

● E. coli HB101 [pEXTNF1] culture supernatant

■ E. coli HB101 [pEXTNF3] culture supernatant

◆ E. coli HB101 [pEXTNF4] culture supernatant

28/34

0317649

# Fig. 25

Culture supernatant of <u>Escherichia coli</u> containing pEXTNF3

Culture supernatant of <u>Escherichia coli</u> containing pEAP8

Pre-stained maker for molecular weight

Lysate of <u>Escherichia coli</u> containing pTNF401NN

MW
— 130K
— 75K
— 50K
— 39K
— 27K
— 17K

## Fig. 26A

Tumor-carrying mouse
before administration of
antitumor-active polypeptide

(Black and white photograph)

# Fig. 26 B

Hemorrhagic necrosis tumor-carrying
mouse after administration of
antitumor-active polypeptide

(Black and white photograph)

31/34

Fig. 26C

Tumor-carrying mouse
before administration of
antitumor-active polypeptide

(Color photograph)

## Fig. 26 D

Hemorrhagic necrosis tumor-carrying
mouse after administration of
antitumor-active polypeptide

(Color photograph)

# Fig. 27

# INTERNATIONAL SEARCH REPORT

0317649

International Application No PCT/JP88/00595

## I. CLASSIFICATION OF SUBJECT MATTER (if several classification symbols apply, indicate all) °

According to International Patent Classification (IPC) or to both National Classification and IPC

Int.Cl⁴ C12N15/00, 1/20, C12P21/02, C07K13/00// (C12N
1/20, C12R1:19), (C12P21/02, C12R1:19)

## II. FIELDS SEARCHED

### Minimum Documentation Searched [7]

| Classification System | Classification Symbols |
|---|---|
| IPC | C12N15/00, 1/20, C12P21/00, 21/02, C07K13/00 |

Documentation Searched other than Minimum Documentation
to the Extent that such Documents are Included in the Fields Searched [8]

BIOSIS database

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | Nature, Vol. 312, (1984) D. Pennica et al [Human tumour necrosis factor: precursor structure, expression and homology tolymphotoxin], P.724-729 | 1-17 |
| A | Nature, Vol. 313, (1985) T. Shirai et al [Cloning and expression in Escherichia coli of the gene for human tumor necrosis factor], P.803-806 | 1-17 |
| A | Journal of Bacteriology, (1986) T. Kobayashi et al [Excretion of the Penicillinase of an Alkalophilic Bacillus sp. through the Escherichia coli Outer Membrane Is Caused by Insertional Activation of the Kil Gene in Plasmid pMB9], P.728-732 | 1-17 |
| P | Gene, Vol. 54, (1987), K. Kato et al [Construction of an excretion vector and extracellular production of human growth hormone from Escherichia coli] P.197-202 | 1-17 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| September 5, 1988 (05. 09. 88) | September 19, 1988 (19. 09. 88) |

| International Searching Authority | Signature of Authorized Officer |
|---|---|
| Japanese Patent Office | |

Form PCT ISA 210 (second sheet) January 1985